# EUROPEAN PATENT APPLICATION

(11) **EP 4 393 516 A1**
(43) Date of publication of application: **03.07.2024**
(21) Application number: 22861082.0
(22) Date of filing: 02.08.2022
(51) Int. Cl.: A61K 47/68, A61K 39/395, A61K 41/00, A61K 49/00, A61P 35/00, A61K 31/409

(54) **ANTIBODY FRAGMENT SPECIFIC TO C-KIT POSITIVE TUMORS**

(30) Priority: 27.08.2021 JP 2021139380
(71) Applicant: Tokushima University, Tokushima-shi, Tokushima 770-8501 (JP)
(72) Inventor: SEITO, Tsutomu, Fujioka-shi, Gunma 375-0005 (JP); SEGAWA, Tatsuya, Fujioka-shi, Gunma 375-0005 (JP); SHIMIZU, Mamoru, Fujioka-shi, Gunma 375-0005 (JP); TAKAYAMA, Tetsuji, Tokushima-shi, Tokushima 770-8501 (JP); MUGURUMA, Naoki, Tokushima-shi, Tokushima 770-8501 (JP); FUJIMOTO, Shota, Tokushima-shi, Tokushima 770-8501 (JP); KASHIHARA, Takanori, Tokushima-shi, Tokushima 770-8501 (JP)
(74) Representative: Gill Jennings & Every LLP
(86) International application number: PCT/JP2022/029636
(87) International publication number: WO 2023/026791

(57) **Abstract**

The purpose of the present invention is to provide a detecting agent (diagnostic agent) and a treatment agent that are taken into tumors in vivo promptly and that bind to c-KIT molecules. In the quest for a molecular species that specifically binds to c-KIT, and a labelling agent and a treatment agent that bind to said molecular species, the inventors found that by combining an anti-c-KIT antibody fragment and IR700, rapid accumulation into tumors takes place in just several hours after administration, and detection and treatment becomes possible. Accordingly, the present invention pertains to an antigen-binding fragment of an anti-c-KIT antibody, IR700, and a composite body, and also pertains to a detecting agent and a treatment agent that contain said composite body as an active ingredient. Specifically, the present invention pertains to: a complementary determining region (CDR) in the antibody heavy chain which has the amino acid sequence described in SEQ ID NO: 2; and a composite body having IR700 and an antigen-binding fragment of an antibody having a CDR in the antibody light chain which has the amino acid sequence described in SEQ ID NO: 4.

## Description

### Technical Field

The present invention relates to the field of diagnosing and treating gastrointestinal stromal tumor, which is a c-KIT-positive tumor.

### Background Art

Gastrointestinal stromal tumor (GIST) is a tumor from mesenchymal cells under the mucosa of the gastrointestinal tract. As the tumor is usually covered by normal mucosa, it can hardly be distinguished from other types of submucosal tumors using standard endoscopy.

Endoscopic ultrasound-guided fine needle aspiration (EUS-FNA) cytology has recently been developed, making it possible to diagnose GIST. However, this requires highly advanced technology, and it is difficult to perform even EUS-FNS, especially when the tumor size is 2 cm or less. Thus, the condition is such that follow-up is performed over time while a histological diagnosis remains impossible. In addition, it has been reported that liver metastasis and rapid tumor growth are observed even in GIST having a size of 2 cm or less. More simplified and effective diagnostic techniques and therapeutic methods are eagerly awaited.

Positron emission tomography (PET) is also used for diagnosing GIST or the like. However, PET is difficult to distinguish GIST or the like from other malignant tumors, including lymphoma and leiomyosarcoma, and inflammatory lesions, and there is also the problem that patients are exposed to high doses of radiation.

Further, in photodynamic therapy (PDT), attempts have been made to treat various types of cancer using photosensitizers that are activated by specific wavelengths of light. While it kills tumor cells, it also damages non-cancerous cells, causing severe side effects.

Meanwhile, laparoscopy was developed using the target molecule c-KIT of GIST by binding Alexa Fluor 488 (AF488) to an anti-c-KIT antibody and using a GIST model mouse. However, this plan was not put into practical use because the autofluorescence of normal tissue was excessively strong, resulting in an overly high false positive rate.

Such a situation led to the idea of using near-infrared (NIR) light. NIR is the most appropriate strategy for endoscopic molecular imaging of GIST because it has very low autofluorescence in living organisms and can penetrate deeper into tissues than visible light. In recent years, photoimmunotherapy (PIT), which enables diagnosis and treatment by binding a photosensitizer to an antibody against a tumor-specific molecule, has been discovered. This is a new type of use of PDT with high selectivity reported. This method is a cancer therapy that minimizes side effects because it only damages cells (tumor cells) that react only with a conjugate of a specific antibody and a dye. The key to this therapy is antibodies specific to molecules that target cancer cells.

It is known that tumor cells of GIST express c-KIT on their surfaces, and 90% or more of patients are positive. Therefore, the present inventors attempted to use c-KIT as a target molecule, couple an IR700 dye to an antibody against this molecule, and diagnose and treat GIST by near-infrared irradiation (Non Patent Literature 1). However, this method requires a long time from administration to detection, such as 24 hours needed for fluorescence to reach its maximum value, i.e., antibodies to accumulate, and 120 hours for the S/N ratio to reach its maximum value, which has been problematic. Further, the research using 90Y-labeled c-KIT antibodies has been reported, and in this case, it also took several days to produce a treatment effect (Non Patent Literature 2). Moreover, previous attempts have been made to label 64Cu using Fab fragments of anti-c-KIT antibodies, but experiments using nude mice have shown that it takes from 6 to 12 hours for uptake (Non Patent Literature 3, Table 3).

### Citation List

### Non Patent Literature

Non Patent Literature 1: Shota Fujimoto et al., Theranostics (2018)8(9):2313-2328.
Non Patent Literature 2: Chisato Yoshida et al., PLOS ONE(2013)8(3)e59248.
Non Patent Literature 3: Chisato Yoshida et al., Nuclear Medicine and Biology(2011)38:331-337.

### Summary of Invention

Accordingly, an object of the present invention is to provide a labeling agent and a treatment agent targeting c-KIT molecules, which are taken up into tumors early *in vivo.*

As a result of examining a molecular species that specifically binds to c-KIT and a labeling agent and a treatment agent which are coupled to the molecular species, the present inventors found that combining the Fab fragment or F(ab')₂ fragment of an anti-c-KIT antibody and IR700 makes it possible to accumulate the combined product in a tumor for a short period, such as several hours from administration for detecting the tumor.

The present inventors also found that PIT treatment is performed after allowing a complex of the Fab fragment or F(ab')₂ fragment of an anti-c-KIT antibody and IR700 to accumulate in a tumor, making it possible also to perform treatment using the same complex as in detection.

Therefore, the present invention relates to a complex of the antigen-binding fragment of an anti-c-KIT antibody and IR700 and also relates to a detection agent and a treatment agent containing the complex as an active ingredient.
(1) A complex comprising: an antigen-binding fragment of an antibody having a complementarity determining region (CDR) in an antibody heavy chain having an amino acid sequence set forth in SEQ ID NO: 2 or SEQ ID NO: 19 and a CDR in an antibody light chain having an amino acid sequence set forth in SEQ ID NO: 4 or SEQ ID NO: 21; and IR700.
(2) The complex according to (1), wherein the antigen-binding fragment of the antibody is Fab or F(ab')₂.
(3) The complex according to (1) or (2), wherein the CDR in the antibody heavy chain having the amino acid sequence set forth in SEQ ID NO: 2 is the following (a) or (b):
   (a) heavy chain CDR (CDRH) 1 consisting of an amino acid sequence set forth in SEQ ID NO: 7,
      CDRH2 consisting of an amino acid sequence set forth in SEQ ID NO: 8, and
      CDRH3 consisting of an amino acid sequence set forth in SEQ ID NO: 9; or
   (b) CDRH1 consisting of an amino acid sequence set forth in SEQ ID NO: 13,
      CDRH2 consisting of an amino acid sequence set forth in SEQ ID NO: 14, and
      CDRH3 consisting of an amino acid sequence set forth in SEQ ID NO: 15.
(4) The complex according to (3), wherein the CDR in the antibody light chain having the amino acid sequence set forth in SEQ ID NO: 4 is the following (a') or (b'):
   (a') light chain CDR (CDRL) 1 consisting of an amino acid sequence set forth in SEQ ID NO: 10,
      CDRL2 consisting of an amino acid sequence set forth in SEQ ID NO: 11, and
      CDRL3 consisting of an amino acid sequence set forth in SEQ ID NO: 12; or
   (b') CDRL1 consisting of an amino acid sequence set forth in SEQ ID NO: 16,
      CDRL2 consisting of amino acid sequence GIS, and
      CDRL3 consisting of an amino acid sequence set forth in SEQ ID NO: 17.
(5) The complex according to any one of (1) to (4), wherein the number of IR700 molecules bound per molecule of the antigen-binding fragment of the antibody (F/P ratio) is from 1 to 10.
(6) The complex according to any one of (1) to (5), wherein the antibody fragment is labeled by further being bound to another labeling substance.
(7) A composition comprising a plurality of the complex according to any one of (1) to (6), wherein an average number of IR700 molecules bound per molecule of the antigen-binding fragment of the antibody of the complex in the composition (F/P ratio) is from 1 to 10.
(8) A cancer detection composition containing the complex according to any one of (1) to (6) as an active ingredient.
(9) The cancer detection composition according to (8), which is used for detecting cancer within 6 hours from administration of the composition.
(10) A cancer treatment composition containing the complex according to any one of (1) to (6) as an active ingredient.
(11) The diagnostic composition according to (10), which is for cancer diagnosis.
(12) A treatment composition containing the complex according to any one of (1) to (6) as an active ingredient.
(13) The treatment composition according to (12), which is for cancer treatment.
(14) The treatment composition according to (12) or (13), wherein after administering the composition, an area where the composition accumulates is irradiated with near-infrared light.
(15) The treatment composition according to (14), wherein the composition is irradiated with near-infrared light within 1 to 12 hours after administration of the composition.
(16) A composition used for both diagnosis and treatment containing the complex according to any one of (1) to (6) as an active ingredient.
(17) The composition according to (16), which is for cancer diagnosis and treatment.
(18) A method for detecting cancer, comprising administering the complex according to any one of (1) to (6).
(19) A method for treating cancer, comprising administering the complex according to any one of (1) to (6).
(20) The complex according to any one of (1) to (6), which is used for cancer treatment.
(21) Use of the complex according to any one of (1) to (6) in producing a medicament for treating cancer.

### Advantageous Effects of Invention

Since the complex of the present invention accumulates in tumors within a short period of several hours after administration, the time required for tumor detection can be shortened. Further, since the complex of the present invention has the effect of killing a tumor when PIT treatment is performed on the tumor in which the complex has accumulated, it can be used for cancer treatment. In particular, since the same complex can be used for detection and treatment, it is also possible to perform every step from detection to treatment by administering one type of drug.

### Brief Description of Drawings

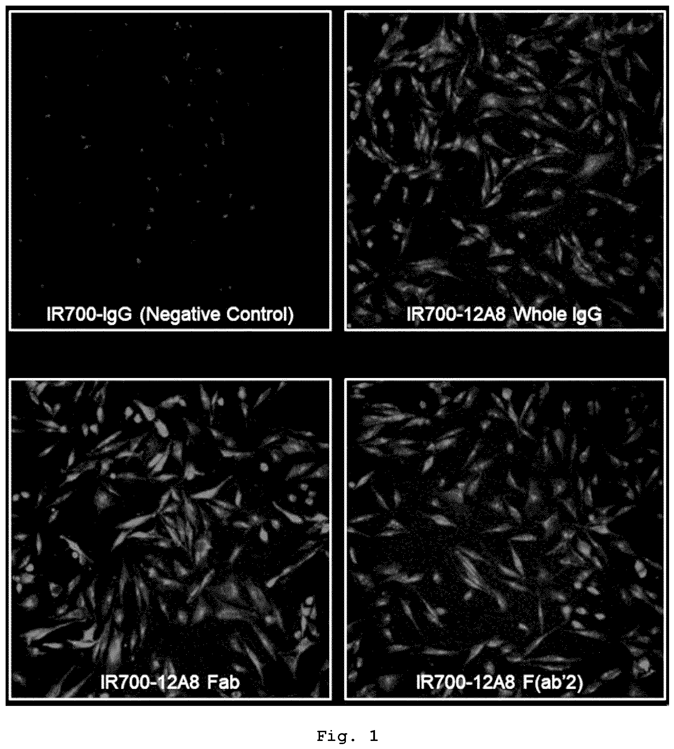

[FIG. 1] FIG. 1 is a view of photographs showing the results of cell imaging using a confocal microscope for each antibody in Example 2. The upper left photo shows the results for IR700-bound IgG (IR700-IgG, negative control). The upper right photo shows the results for the IR700-bound full-length 12A8 antibody (IR700-12A8 Whole IgG). The lower left photo shows the results for the Fab fragment of the IR700-bound 12A8 antibody (IR700-12A8 Fab). The lower right photo shows the results for the F(ab')₂ fragment of the IR700-bound 12A8 antibody (IR700-12A8 F(ab')₂).

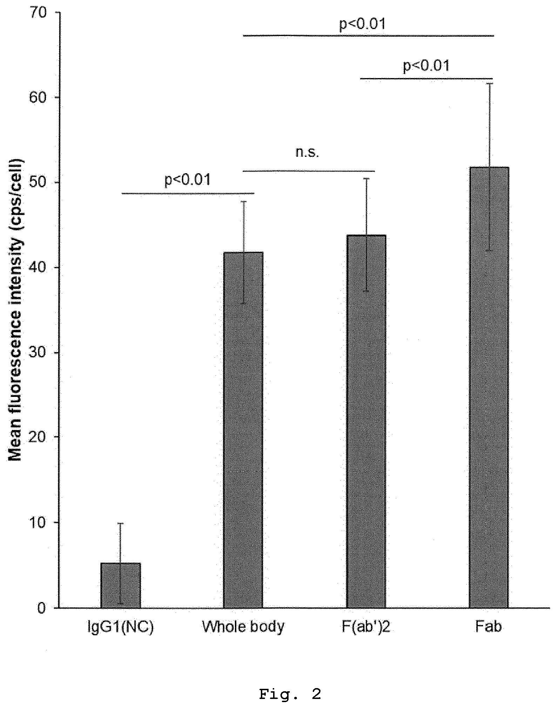

[FIG 2] FIG. 2 is a graph showing the fluorescence intensity measured in FIG. 1. The vertical axis shows the average fluorescence intensity (cps/cell). IgG1(NC) shows the results for negative control IgG. Whole body shows the results for the full-length 12A8 antibody. F(ab')₂ shows the results for the F(ab')₂ fragment of the 12A8 antibody. Fab shows the results for the Fab fragment of the 12A8 antibody.

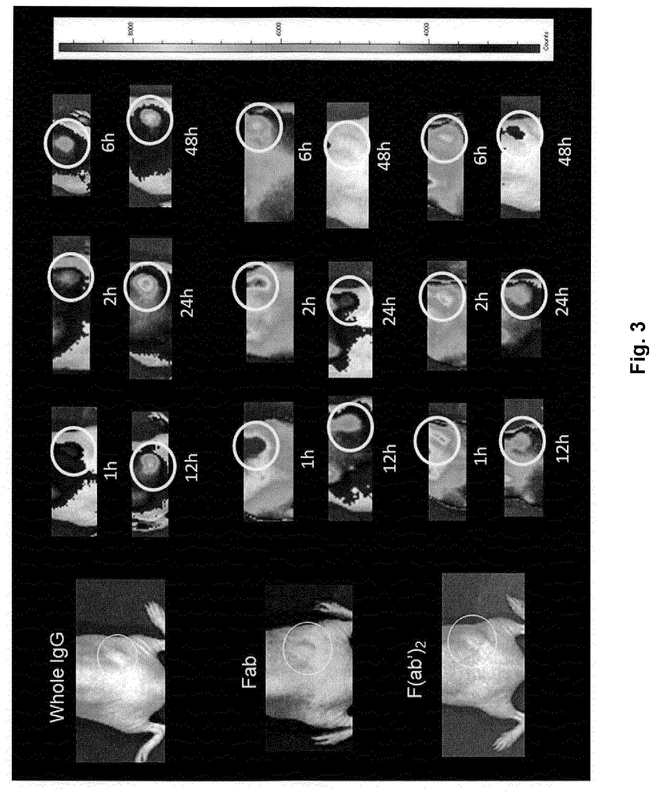

[FIG. 3] FIG. 3 shows the fluorescence imaging results of each antibody in a cancer-bearing nude mouse model. Whole IgG shows the results for the full-length 12A8 antibody. F(ab')₂ shows the results for the F(ab')₂ fragment of the 12A8 antibody. Fab shows the results for the Fab fragment of the 12A8 antibody. The elapsed time (h) from administration of the labeled antibody or labeled antigen-binding fragment is shown at the bottom of each photograph.

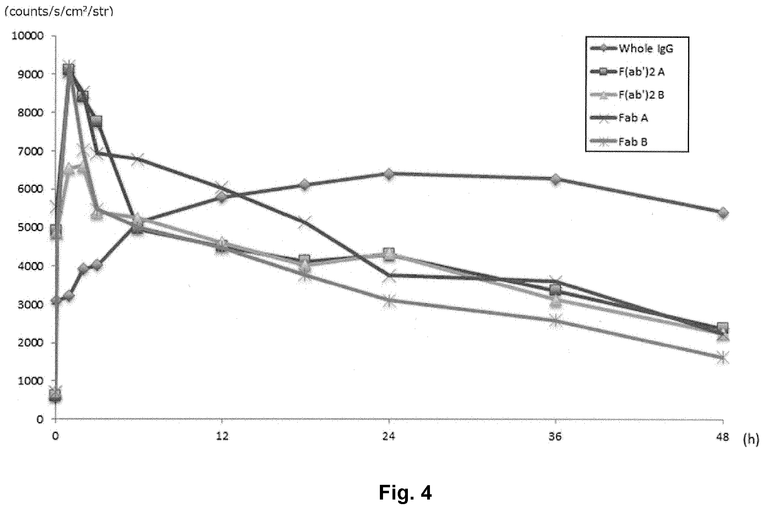

[FIG 4] FIG. 4 is a graph showing time-dependent change in the fluorescence intensity measured in FIG. 3. The vertical axis represents the fluorescence intensity (counts/s/cm²/str), and the horizontal axis represents the elapsed time (h) from administration of the labeled antibody or labeled antigen-binding fragment.

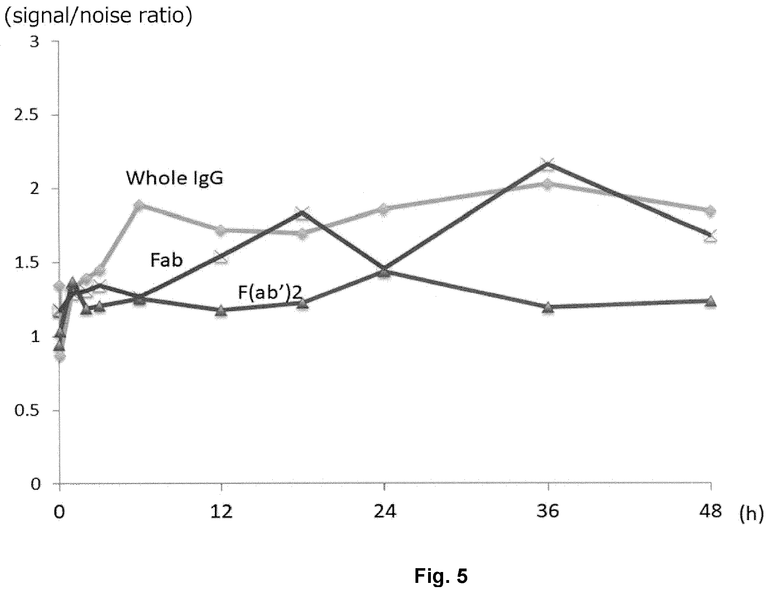

[FIG. 5] FIG. 5 is a graph showing the S/N ratio of each antibody in a cancer-bearing nude mouse model. The vertical axis represents the signal-to-noise ratio, and the horizontal axis represents the elapsed time (h) from administration of the labeled antibody or labeled antigen-binding fragment.

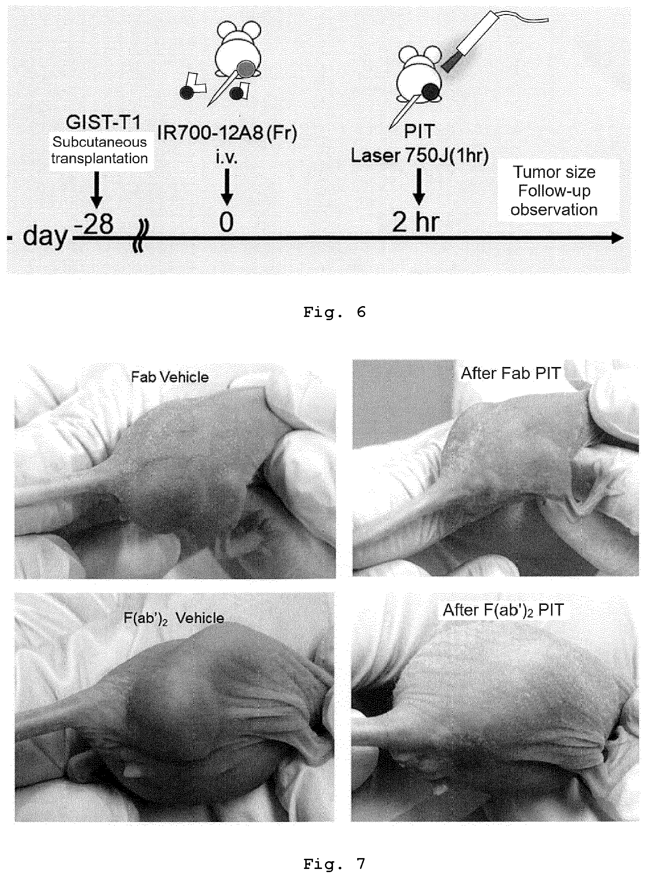

[FIG. 6] FIG. 6 is a schematic diagram of an *in vivo* photoimmunotherapy (PIT) treatment test.
[FIG. 7] FIG. 7 is a view of photographs comparing before and after PIT treatment using cancer-bearing nude mice. The left photos show negative control mice without PIT treatment: mouse administered with the 12A8-antibody Fab fragment (Fab Vehicle) (upper) and mouse administered with the 12A8-antibody F(ab')₂ fragment (F(ab')₂ Vehicle) (lower). The right photos show mice of the PIT treatment group: mouse of 12A8-antibody Fab fragment administration + PIT treatment (after Fab PIT) (upper) and mouse of 12A8-antibody F(ab')₂ fragment administration + PIT treatment (after F(ab')₂ PIT) (lower).

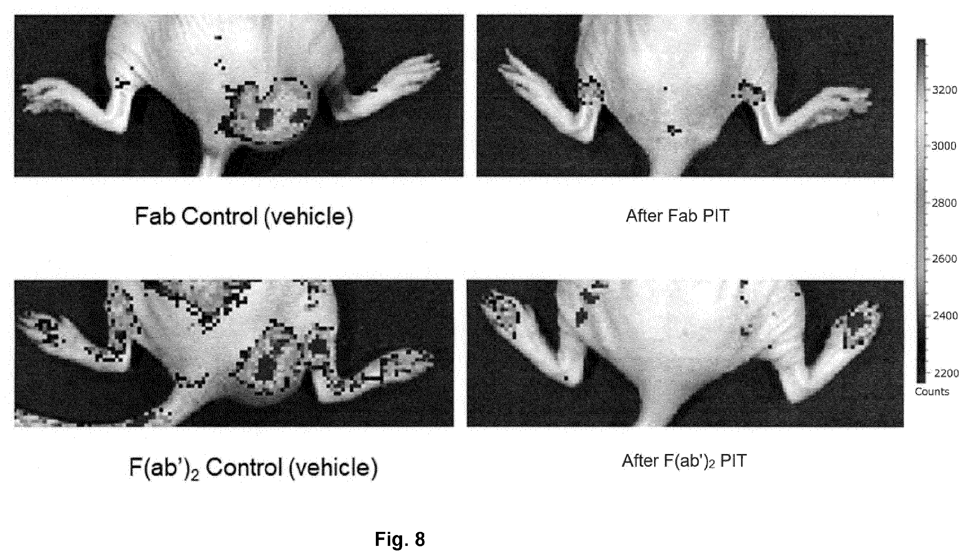

[FIG. 8] FIG. 8 is a view of photographs comparing the fluorescence intensity of the fluorescently labeled anti-c-KIT antibody before and after PIT treatment using cancer-bearing nude mice. The left photos show negative control mice without PIT treatment: mouse treated with the 12A8-antibody Fab fragment (Fab Control(Vehicle)) (upper) and mouse treated with the 12A8-antibody F(ab')₂ fragment (F(ab')₂ Control(Vehicle)) (lower). The right photos show mice of the PIT treatment group: mouse of 12A8-antibody Fab fragment administration + PIT treatment (after Fab PIT) (upper) and mouse of 12A8-antibody F(ab')₂ fragment administration + PIT treatment (after F(ab')₂ PIT) (lower).

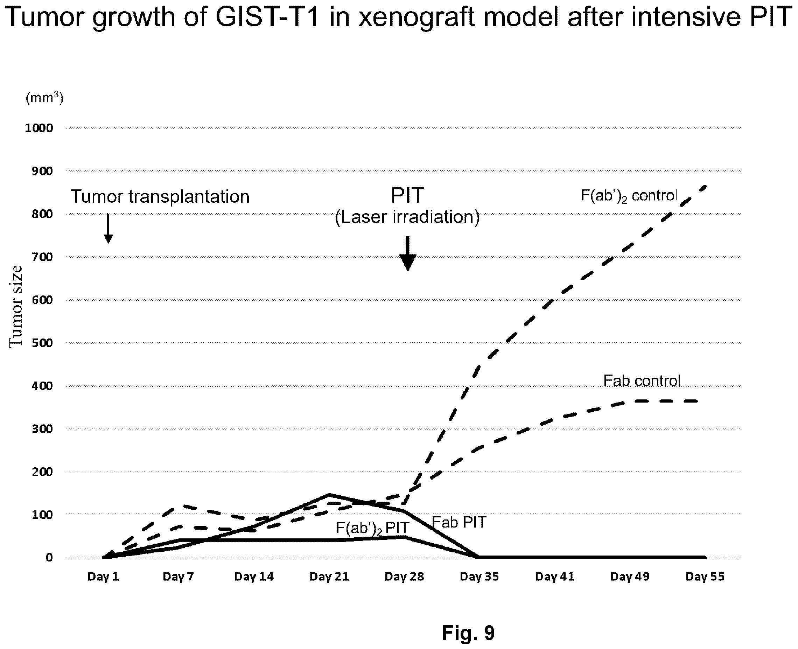

[FIG. 9] FIG. 9 is a graph showing time-dependent changes in tumor size in the PIT treatment test using cancer-bearing nude mice. The vertical axis represents the tumor size (mm³), and the horizontal axis represents the number of days elapsed after tumor transplantation. PIT treatment was performed 28 days after tumor transplantation.

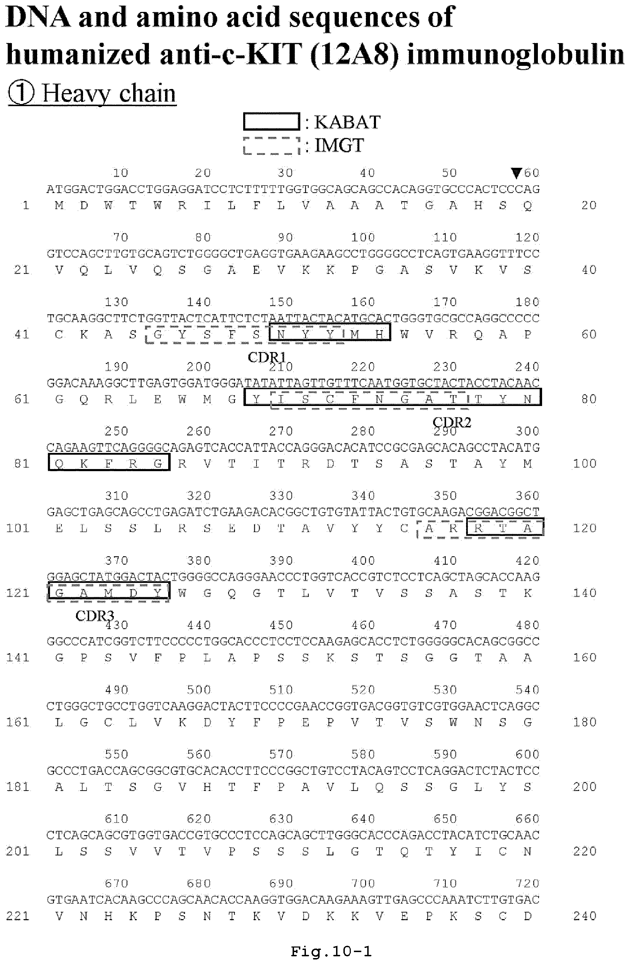

[FIG. 10-1] FIG. 10-1 shows DNA and amino acid sequences of the heavy chain of the humanized anti-c-KIT (12A8) antibody.

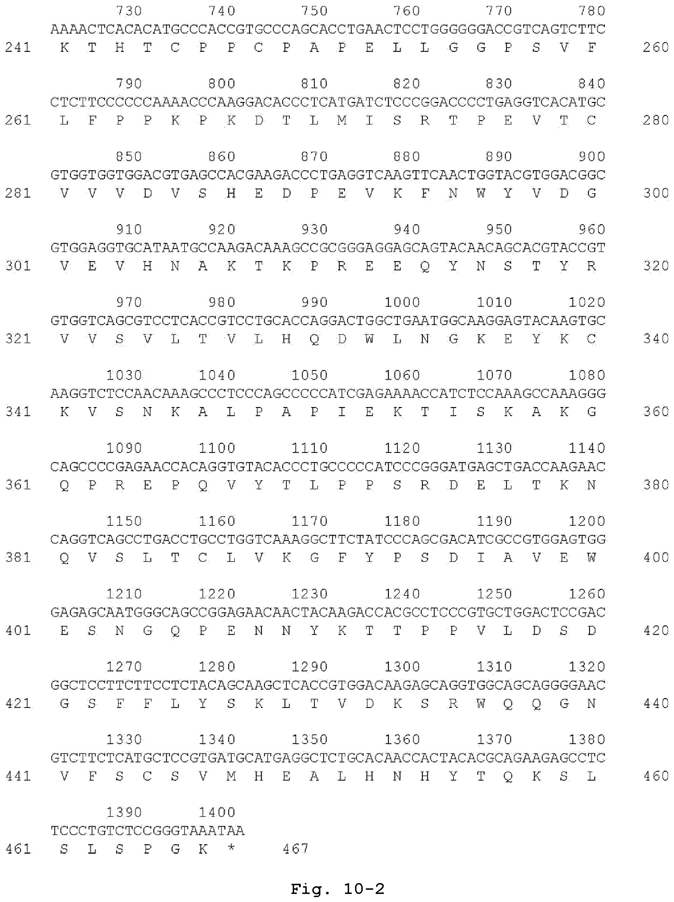

[FIG. 10-2] FIG. 10-2 shows DNA and amino acid sequences of the heavy chain of the humanized anti-c-KIT (12A8) antibody.

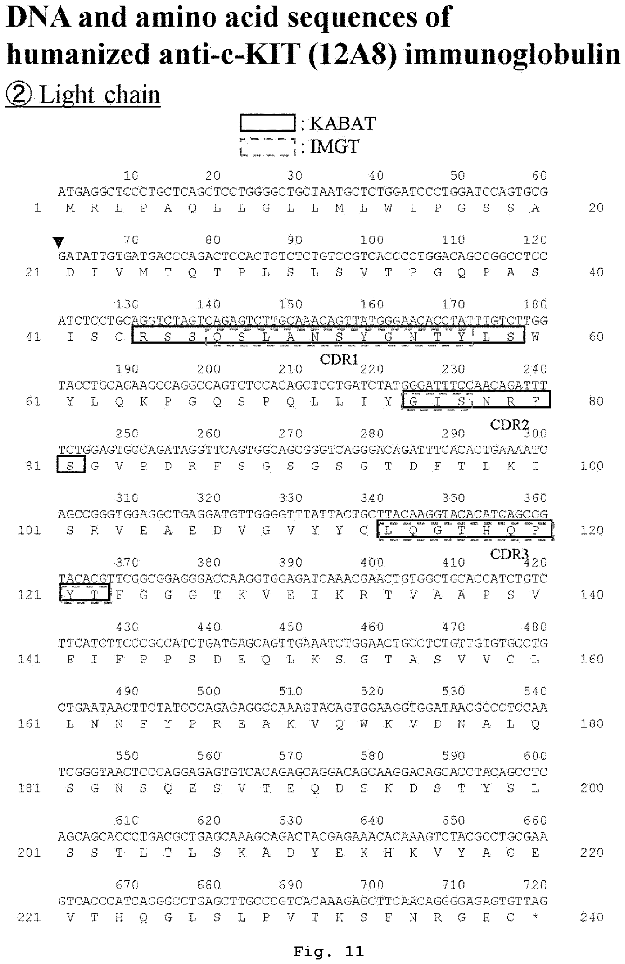

[FIG. 11] FIG. 11 shows DNA and amino acid sequences of the light chain of the humanized anti-c-KIT (12A8) antibody.

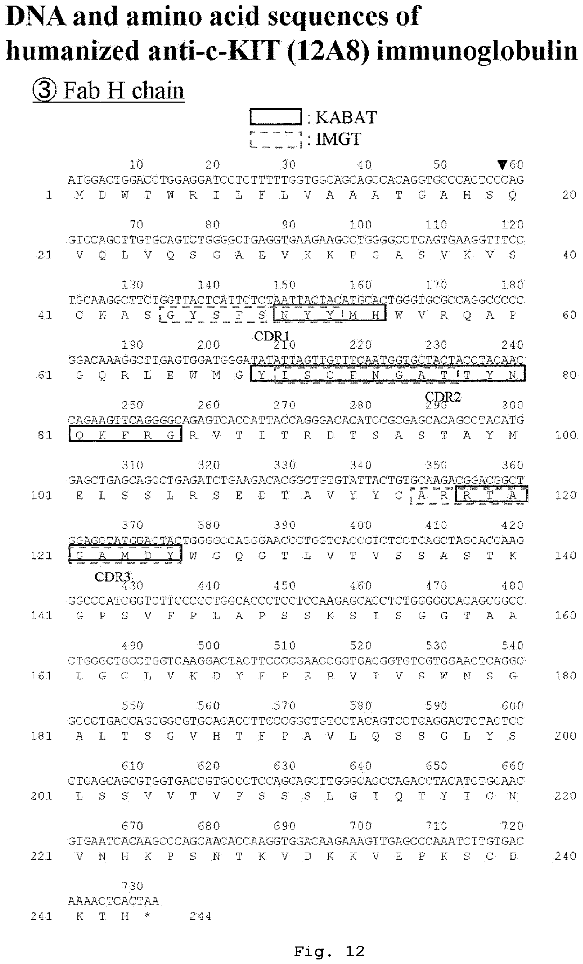

[FIG. 12] FIG. 12 shows DNA and amino acid sequences of the Fab H chain of the humanized anti-c-KIT (12A8) antibody.

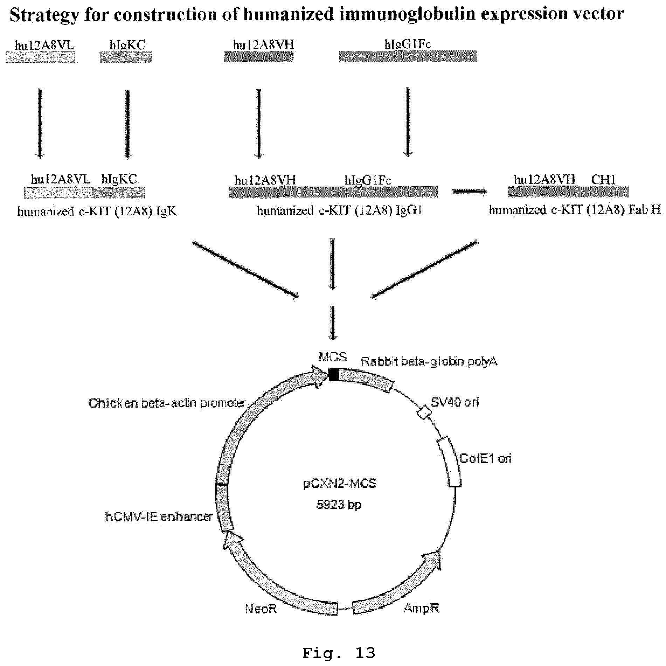

[FIG. 13] FIG. 13 shows the structure of the humanized anti-c-KIT (12A8) antibody-expressing vector.

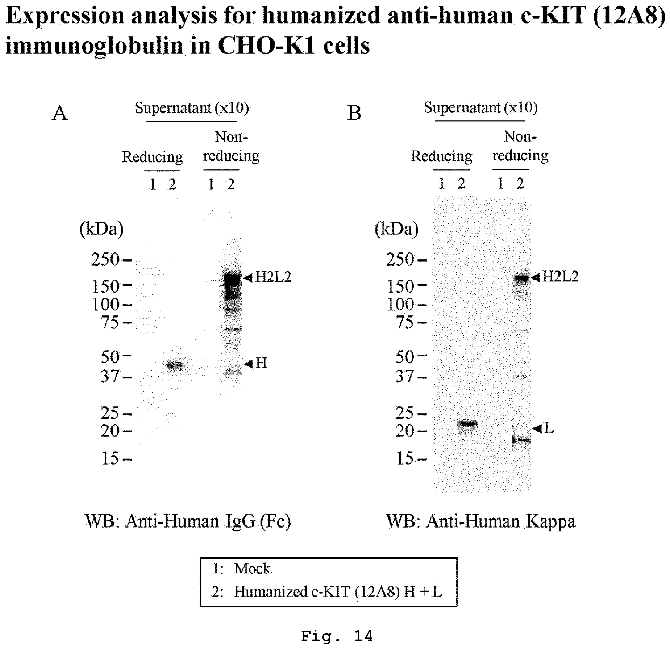

[FIG. 14] FIG. 14 is a view of Western blot photographs showing the expression analysis results for the humanized anti-c-KIT (12A8) IgG1 antibody in CHO-K1 cells. Mock (Lane 1) and the humanized anti-c-KIT (12A8) IgG1 antibody (Lane 2) were used as primary antibodies. The HRP-labeled anti-human IgG(Fc) antibody was used in A and the HRP-labeled anti-human Kappa antibody was used in B as secondary antibodies.

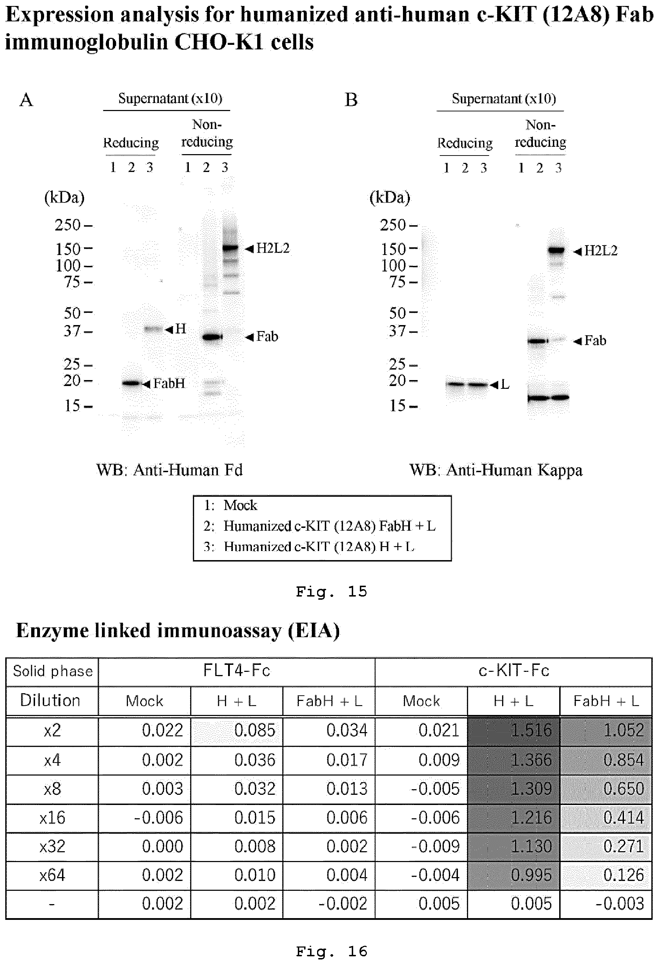

[FIG. 15] FIG. 15 is a view of Western blot photographs showing the expression analysis results for the humanized anti-c-KIT (12A8) Fab antibody in CHO-K1 cells. Mock (Lane 1), the humanized anti-c-KIT (12A8) Fab antibody (Lane 2), and the humanized anti-c-KIT (12A8) IgG1 antibody (Lane 3) were used as primary antibodies. The HRP-labeled anti-human Fd antibody was used in A and the HRP-labeled anti-human Kappa antibody was used in B as secondary antibodies.
[FIG. 16] FIG. 16 shows the reactivity of the humanized anti-c-KIT (12A8) IgG1 antibody and the humanized anti-c-KIT (12A8) Fab antibody confirmed by EIA.

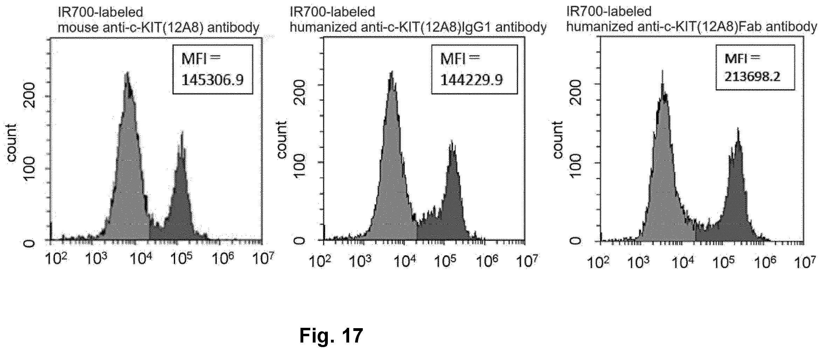

[FIG. 17] FIG. 17 shows the reactivity of the IR700-labeled humanized anti-c-KIT (12A8) antibodies (whole IgG and Fab) confirmed by flow cytometry.

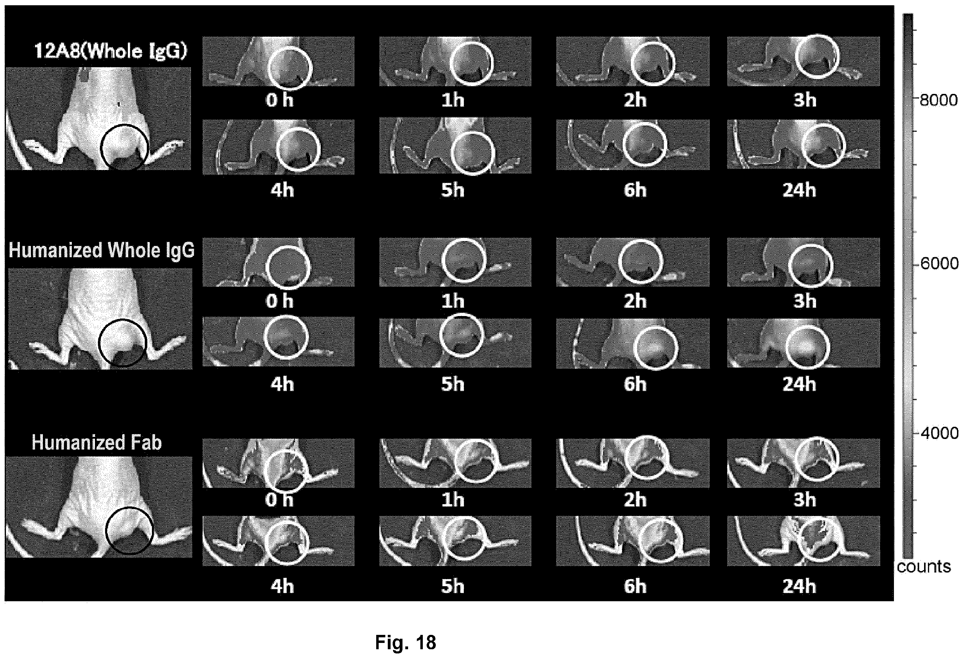

[FIG. 18] FIG. 18 is an image of photos showing the fluorescence imaging results of each antibody in a cancer-bearing nude mouse model. 12A8 (whole IgG) refers to the IR700-labeled humanized anti-c-KIT (12A8) antibody. Humanized whole IgG refers to the anti-c-KIT (12A8) IgG1 antibody. Humanized Fab refers to the anti-c-KIT (12A8) Fab antibody. The elapsed time (h) from administration of the labeled antibody or labeled antigen-binding fragment is shown at the bottom of each photograph.

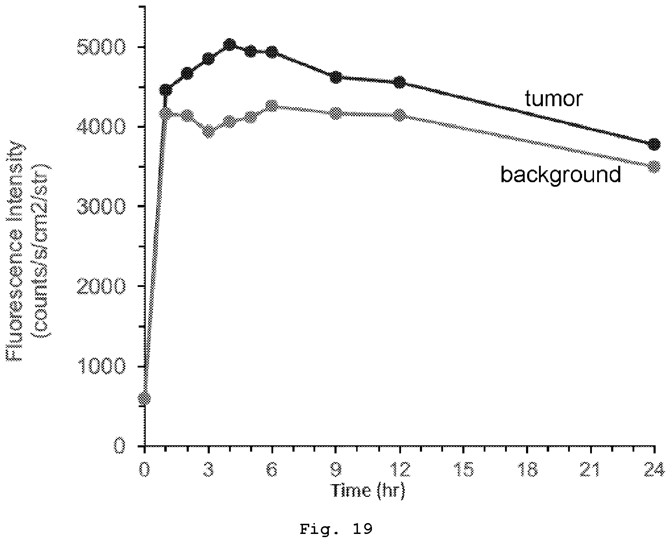

[FIG. 19] FIG. 19 shows time-dependent changes in the fluorescence intensity of xenograft tumors in mice caused by the IR700-labeled humanized anti-c-KIT (12A8) Fab antibody. The vertical axis represents the fluorescence intensity, and the horizontal axis represents the elapsed time (h) from administration of the labeled antigen-binding fragment.

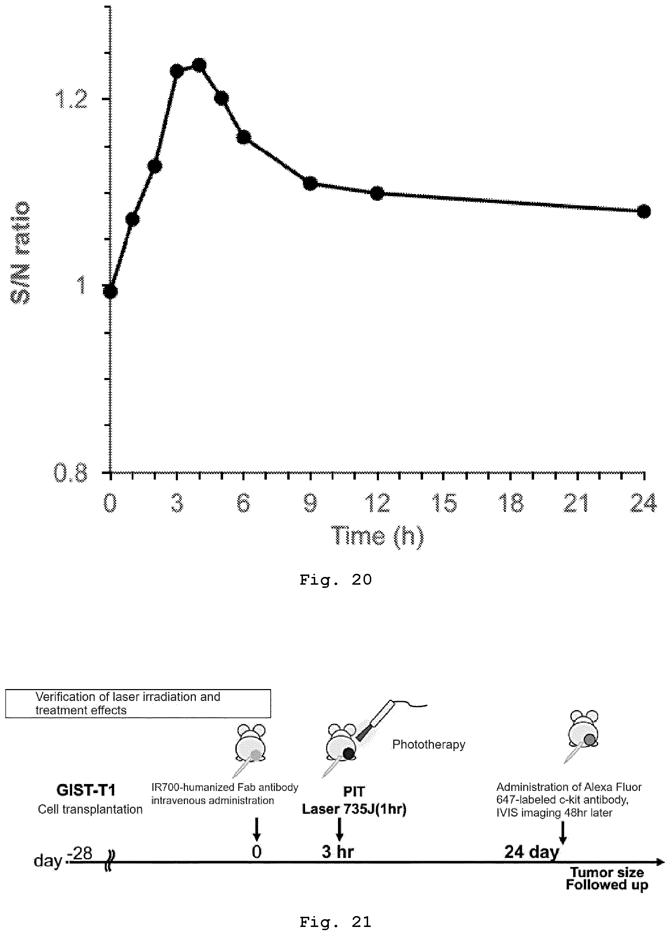

[FIG. 20] FIG. 20 shows time-dependent changes in the S/N ratio in the mouse tumor region when using the IR700-labeled humanized anti-c-KIT (12A8) Fab antibody. The vertical axis represents the S/N ratio, and the horizontal axis represents the elapsed time (h) from administration of the labeled antigen-binding fragment.
[FIG. 21] FIG. 21 is a schematic diagram of the photoimmunotherapy schedule using cancer-bearing mice.

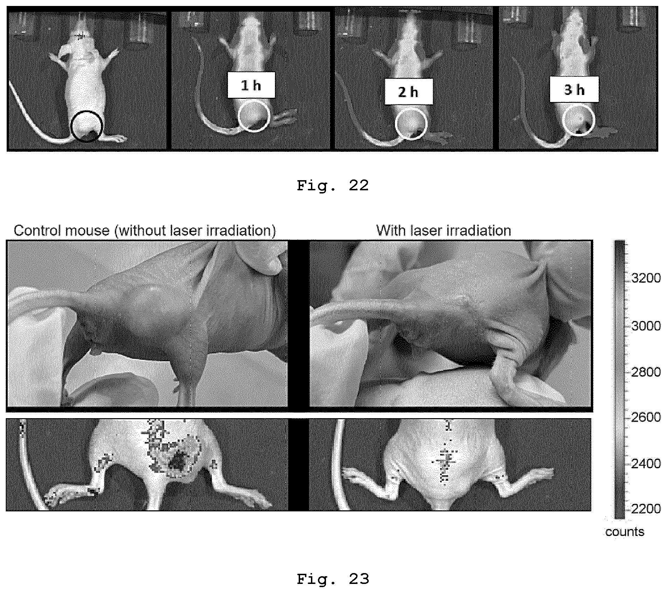

[FIG. 22] FIG. 22 is a view of photographs showing fluorescence imaging of tumors using the IR700-labeled humanized anti-c-KIT (12A8) Fab antibody.
[FIG. 23] FIG. 23 is a view of photographs showing fluorescence imaging indicating the effects of photoimmunotherapy using the IR700-labeled humanized anti-c-KIT (12A8) Fab antibody.

### Description of Embodiments

### (Antigen-Binding Fragment)

The antigen-binding fragment described herein is an antibody fragment having a complementarity determining region (CDR) in the antibody heavy chain having the amino acid sequence set forth in SEQ ID NO: 2 or SEQ ID NO: 19 and a CDR in the antibody light chain having the amino acid sequence set forth in SEQ ID NO: 4 or SEQ ID NO: 21. SEQ ID NOS: 2 and 4 each set forth the amino acid sequence of a mouse-derived antigen-binding fragment. SEQ ID NOS: 19 and 21 each set forth the amino acid sequence of a humanized antigen-binding fragment.

The amino acid sequence set forth in SEQ ID NO: 2 may be encoded by, for example, the base sequence set forth in SEQ ID NO: 1. The amino acid sequence set forth in SEQ ID NO: 4 may be encoded by, for example, the base sequence set forth in SEQ ID NO: 3. The amino acid sequence set forth in SEQ ID NO: 19 may be encoded by, for example, the base sequence set forth in SEQ ID NO: 18. The amino acid sequence set forth in SEQ ID NO: 21 may be encoded by, for example, the base sequence set forth in SEQ ID NO: 20. In addition, the antigen-binding fragment may have a complementarity determining region in the Fab H chain having the amino acid sequence set forth in SEQ ID NO: 6; the amino acid sequence set forth in SEQ ID NO: 6 may be encoded by, for example, the base sequence set forth in SEQ ID NO: 5. In addition, the antigen-binding fragment may have a complementarity determining region in the Fab H chain having the amino acid sequence set forth in SEQ ID NO: 23; the amino acid sequence set forth in SEQ ID NO: 23 may be encoded by, for example, the base sequence set forth in SEQ ID NO: 22. Reported examples of a method for determining a CDR used herein include the methods of Kabat et al., Chothia et al., Martin et al., Geldand et al., IMGT (registered trademark) (http://www.imgt.org/IMGTindex/CDR.php), and Honnerger et al. (AHo's) (Lars Nieba et al., Protein Engineering, 10(4):435-444(1997)). A CDR may be determined based on any definition as long as an antibody fragment having the CDR specifically binds to c-KIT.

Examples of the heavy chain CDR in the antigen-binding fragment described herein can include: CDRH1 consisting of the amino acid sequence set forth in SEQ ID NO: 7, CDRH2 consisting of the amino acid sequence set forth in SEQ ID NO: 8, and CDRH3 consisting of the amino acid sequence set forth in SEQ ID NO: 9; or CDRH1 consisting of the amino acid sequence set forth in SEQ ID NO: 13, CDRH2 consisting of the amino acid sequence set forth in SEQ ID NO: 14, and CDRH3 consisting of the amino acid sequence set forth in SEQ ID NO: 15. Examples of the light chain CDR in the antigen-binding fragment described herein can include: CDRL1 consisting of the amino acid sequence set forth in SEQ ID NO: 10, CDRL2 consisting of the amino acid sequence set forth in SEQ ID NO: 11, and DRL3 consisting of the amino acid sequence set forth in SEQ ID NO: 12; or CDRL1 consisting of the amino acid sequence set forth in SEQ ID NO: 16, CDRL2 consisting of the amino acid sequence GIS (i.e., Gly, Ile, Ser), and CDRL3 consisting of the amino acid sequence set forth in SEQ ID NO: 17. For example, the antigen-binding fragment described herein may have: (i) CDRH1 consisting of the amino acid sequence set forth in SEQ ID NO: 7, CDRH2 consisting of the amino acid sequence set forth in SEQ ID NO: 8, CDRH3 consisting of the amino acid sequence set forth in SEQ ID NO: 9, CDRL1 consisting of the amino acid sequence set forth in SEQ ID NO: 10, CDRL2 consisting of the amino acid sequence set forth in SEQ ID NO: 11, and DRL3 consisting of the amino acid sequence set forth in SEQ ID NO: 12; or (ii) CDRH1 consisting of the amino acid sequence set forth in SEQ ID NO: 13, CDRH2 consisting of the amino acid sequence set forth in SEQ ID NO: 14, CDRH3 consisting of the amino acid sequence set forth in SEQ ID NO: 15, CDRL1 consisting of the amino acid sequence set forth in SEQ ID NO: 16, CDRL2 consisting of the amino acid sequence GIS, and CDRL3 consisting of the amino acid sequence set forth in SEQ ID NO: 17.

The term "antigen-binding fragment" used herein refers to a fragment of an antibody, the fragment retaining the antigen-binding ability of the antibody. Examples of the antigen-binding fragment can include F(ab')₂, Fab', Fab, Fabs, single-chain Fv (hereinafter referred to as "scFv"), (tandem) bispecific single-chain Fv(sc(Fv)₂), single-chain triple body, nanobody, divalent VHH, pentavalent VHH, minibody, (double-chain) diabody, tandem diabody, bispecific tribody, bispecific bibody, dual-affinity re-targeting (DART) molecule, triabody (or tribody), tetrabody (or [sc(Fv)₂]₂ or (scFv-SA)₄) disulfide-bond Fv (hereinafter referred to as "dsFv"), compact IgG, heavy chain antibody, and polymers thereof.

The antigen-binding fragment that forms part of the complex of the present invention specifically binds to c-KIT. The expression "antigen-binding fragment "specifically" recognizes (bind)" used herein means that the antigen-binding fragment binds to c-KIT with substantially higher affinity than to other proteins or peptides. The expression "binds to... with substantially higher affinity than to" used herein means that high affinity to the extent that the specific protein or peptide of interest can be detected separately from other proteins or peptides using the desired measurement device or method. For example, "substantially higher affinity" may mean 3 times or more, 4 times or more, 5 times or more, 6 times or more, 7 times or more, 8 times or more, 9 times or more, 10 times or more, 20 times or more, 30 times or more, 40 times or more, 50 times or more, or 100 times or more affinity in terms of the intensity detected by ELISA or EIA (e.g., fluorescence intensity).

As used herein, the binding rate constant (Ka1) for the binding between antigen-binding fragment and c-KIT can be, for example, 1 × 10⁴ Ms⁻¹ or more, 1 × 10⁵ Ms⁻¹ or more, or 5 × 10⁵ Ms⁻¹ or more. The dissociation rate constant (Kd1) for the binding between the antigen-binding fragment and c-KIT includes, for example, 1 × 10⁻³ or less or 1 × 10⁻⁴ or less. The binding constant (KD) for the binding between the antigen-binding fragment and c-KIT is, for example, 1 × 10⁻⁸ (M) or less, 5 × 10⁻⁸ (M) or less, 1 × 10⁻⁹ (M) or less, or 5 × 10⁻⁹ (M) or less. For the binding rate constant (Ka1), dissociation rate constant (Kd1), and binding constant (KD) of the antigen-binding fragment described herein, BIACORE (GE Healthcare Bioscience Holding Limited, BIACORE-X100) is used. Biotinylated c-KIT is immobilized on the SA chip, and then the test antibody is poured thereon according to the manufacturer's manual. Accordingly, the binding rate constant Ka1 and dissociation rate constant Kd1 can be measured, and the binding constant KD value can be determined using bivalent fitting.

The antigen-binding fragment includes an antigen-binding fragment of a non-human animal and an antigen-binding fragment having an amino acid sequence of a non-human animal and a human-derive amino acid sequence. For example, the antigen-binding fragment may be a humanized antigen-binding fragment. A humanized antigen-binding fragment is a fragment of an antibody in which the genes encoding the H chain and L chain of a human antibody are used as bases, and the primary structural parts of the six CDRs thereof have been genetically engineered to be replaced by the primary structural parts of the CDRs of the H chain (3 locations) and L chain (3 locations) of an anti-c-KIT antibody from a non-human animal. For example, in the case of using the antibody of the present invention in diagnosis by treatment, prevention, or *in vivo* administration with the antibody, the antibody of the present invention is preferably a humanized antibody fragment.

The complex of the present invention may further contain one or more other different antigen-binding fragments of antibodies; thus, it may be monospecific, bispecific, trispecific, or multispecific.

Amino acids described herein are each represented by a single-letter code. Specifically, A represents alanine, L represents leucine, R represents arginine, K represents lysine, N represents asparagine, M represents methionine, D represents aspartic acid, F represents phenylalanine, C represents cysteine, P represents proline, Q represents glutamine, S represents serine, E represents glutamic acid, T represents threonine, G represents glycine, W represents tryptophan, H represents histidine, Y represents tyrosine, I represents isoleucine, and V represents valine.

IR700 is also referred to as phthalocyanine and means a compound having the following structure.

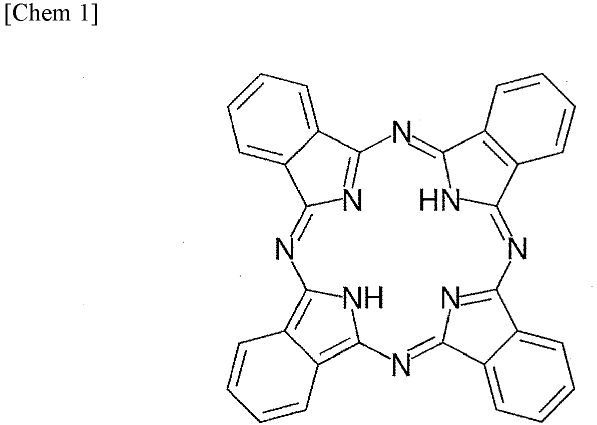

IR700 included in the complex described herein may be a derivative thereof as long as it has the above-described structure. For example, when IR700 has a substituent that reacts with an amino group or a carboxyl group, the antigen-binding fragment and IR700 can be bound by a reaction between the substituent and the amino group or carboxyl group of the antigen-binding fragment. For example, IRDye (registered trademark) 700DX (LI-COR, Inc.) having the following structure can be used herein as IR700. In this case, the antigen-binding fragment and IR700 can be bound according to the protocol provided by the manufacturer.

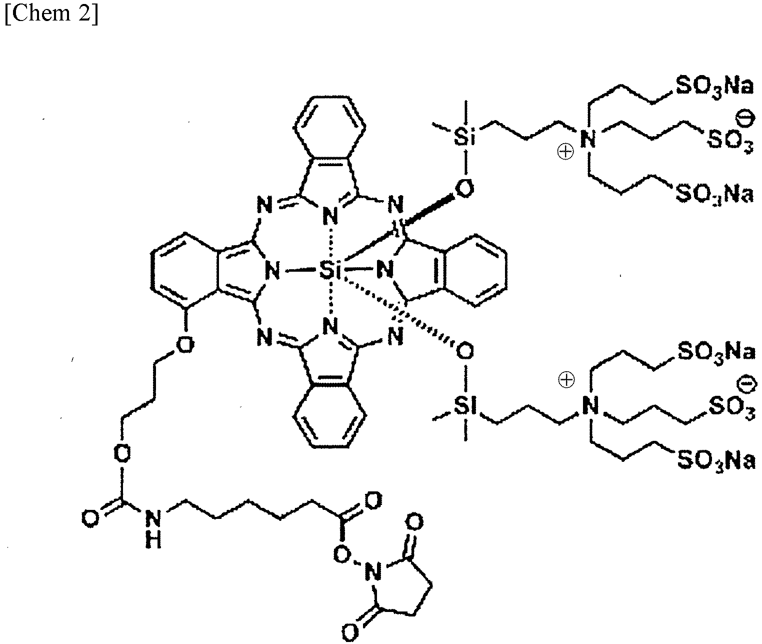

The binding ratio between the antigen-binding fragment and IR700 may be, for example, from 1 to 10, from 1 to 5, or from 1 to 3 in terms of the number of IR700 molecules bound per molecule of the antigen-binding fragment (F/P ratio). In the case of a composition comprising a plurality of the complex of the present invention, the average number of IR700 molecules bound per molecule of the antigen-binding fragment of the antibody of the complex in the composition (F/P ratio) may be from 1 to 10, from 1 to 5, from 1 to 3, or from 1.5 to 3.0.

Since the complex of the present invention has IR700, it can be used for detection and diagnosis by measuring its fluorescence. Therefore, in another aspect, the present invention relates to a cancer detection/diagnosis composition containing the above-described complex as an active ingredient. The present invention also relates to a method for detecting/diagnosing any cancer cells expressing c-KIT, comprising administering the complex of the present invention to a subject having cancer and detecting an area where the complex accumulates in the subject.

As used herein, "cancer" refers to cells that have undergone malignant transformation such that they become pathogens to the host organism. As used herein, cancer includes not only primary cancer but also metastatic cancer and *in vitro* cultures and cell lines from cancer cells. Cancer may be, for example, a hematopoietic tumor, which is a tumor of blood cells. Examples thereof include: leukemia such as chronic myeloid leukemia or acute myeloid leukemia; myeloma such as multiple myeloma; and lymphoma. Examples of cancers include, but are not limited to, lung cancer, non-small cell lung cancer, small cell lung cancer, non-Hodgkin lymphoma, adrenal cortex cancer, AIDS-related cancer, AIDS-related lymphoma, childhood cerebellar astrocytoma, childhood cerebral astrocytoma, basal cell carcinoma, skin cancer (non-melanoma), biliary tract cancer, extrahepatic bile duct cancer, intrahepatic bile duct cancer, bladder cancer, bone and joint cancer, osteosarcoma and malignant fibrous histiocytoma, brain cancer, brain tumor, brain stem glioma, cerebellar astrocytoma, glioma, cerebral astrocytoma/malignant glioma, lining tumor, medullary blastoma, supratentorial primitive neuroectodermal tumor, optic pathway/hypothalamic glioma, head and neck cancer, metastatic squamous epithelial cervical cancer, bronchial adenoma/carcinoid, carcinoid tumor, nervous system lymphoma, central nervous system cancer, central nervous system lymphoma, neuroblastoma, pediatric cancer, Seziary syndrome, extracranial germ cell tumor, extragonadal germ cell tumor, extrahepatic bile duct cancer, eye cancer, intraocular melanoma, retinal blastoma, salivary adenocarcinoma, oral cancer, oral cavity cancer, oropharyngeal cancer, oral cancer, tongue cancer, esophageal cancer, gastric cancer, gastrointestinal carcinoid tumor, gastrointestinal stromal tumor (GIST), small intestinal cancer, colon cancer, colorectal cancer, rectal cancer, anal-rectal cancer, anal cancer, germ cell tumor, hepatocyte (liver) cancer, Hodgkin lymphoma, laryngeal cancer, pharyngeal cancer, hypopharyngeal cancer, nasopharyngeal cancer, sinus and nasal cancer, throat cancer, pancreatic islet cell tumor (endocrine pancreas), pancreatic cancer, parathyroid cancer, liver cancer, gallbladder cancer, appendix cancer, kidney cancer, urinary tract cancer, transitional epithelial cancer of the renal pelvis and urinary tract and other urinary cancer, squamous epithelial cancer, penis cancer, testis cancer, thoracic adenomas, thoracic adenoma and thoracic adenocarcinoma, thyroid cancer, prostate cancer, ovarian cancer, ovarian epithelial cancer, low-grade ovarian tumor, ovarian germ cell tumor, gestational trophoblastic tumor, breast cancer, uterine cancer, uterine sarcoma, cervical cancer, endometrial cancer, uterine body cancer, vaginal cancer, vulval cancer, Wilms tumor, acute lymphoblastic leukemia, acute myeloid leukemia, chronic lymphocytic leukemia, chronic myeloid leukemia, multiple myeloma, chronic myeloid proliferative disease, hairy cell leukemia, primary central nervous system lymphoma, chronic myeloid proliferative disease, cutaneous t-cell lymphoma, lymphoid neoplasm, Waldenstrom macroglobulinemia, medullary blastoma, skin cancer (non-melanoma), skin cancer (melanoma), pheochromocytoma, Merkel cell skin cancer, mesothelioma, malignant mesothelioma, multiple endocrine tumor syndrome, myelodysplastic syndrome, myelodystrophy/myeloid proliferative disease, pineoblastoma and pituitary tumor, plasmacytoma, pleuropulmonaryblastoma, retinal blastoma, rhabdomyosarcoma, sarcoma, a family of Ewing tumors, soft tissue sarcoma, soft tissue sarcoma, mycosis fungoides, and Kaposi's sarcoma. Further, the present invention is also effective in detecting (diagnosing) and treating benign tumors, including precancerous lesions, which express c-KIT.

The complex of the present invention rapidly accumulates in cancer cells after contact with cancer cells or after administration into a living body having cancer. Therefore, the cancer detection composition of the present invention can be administered within 24 hours, 12 hours, 6 hours, 5 hours, 4 hours, 3 hours, 2 hours, or 1 hour after administration of the composition for detecting cancer. From the viewpoint of detection in a short period, it is preferable to use the composition to detect cancer within 4 hours, 3 hours, 2 hours, or 1 hour.

In addition, the complex of the present invention has an IR700 molecule and thus can kill cancer cells with photoimmunotherapy (PIT) (Mitsunaga M et al., Nat Med.(2011)17(12): 1685-91.). Therefore, after the period of administering the cancer treatment composition of the present invention to a subject and allowing the composition to accumulate in the cancer has elapsed, the cancer area (i.e., an area where the composition has accumulated) is irradiated with near-infrared light, thereby allowing the cancer treatment effect to be exerted. Accordingly, the present invention relates to a cancer treatment composition containing the above-described complex as an active ingredient. As used herein, the composition and complex primarily used for cancer treatment are combined with near-infrared light for use. The present invention also relates to a method for treating cancer, comprising administering the complex of the present invention to a subject having cancer and irradiating an area where the complex accumulates in the subject with near-infrared light.

As near-infrared light, light having a wavelength of from 650 to 900 nm, preferably from 650 to 750 nm, more preferably about 700 nm, can be used. The time for accumulating the composition in cancer can be from 1 to 24 hours, 1 to 12 hours, 1 to 6 hours, 2 to 6 hours, 2 to 4 hours, 2 to 3 hours, or 3 to 4 hours later after administration of the composition.

The antibody fragment of the present invention can also be further combined with a labeling substance. For example, it is possible to create a complex by combining it with a fluorescent substance or a photosensitizer having a wavelength in the visible light range, such as from 480 to 650 nm, and apply it to cancer diagnosis and treatment. As the label, detectable labels such as radioactive labels, enzymes, fluorescent labels, bioluminescent labels, chemiluminescent labels, and metals can be used. Examples of such labels can include, but are not limited to: radioactive labels such as 32P, 3H, 125I, 131I, 14C, and tritium; enzymes such as β-galactoxidase, peroxidase, alkaline phosphatase, glucoamylase, glucose oxidase, lactate oxidase, alcohol oxidase, monoamine oxidase, and horseradish peroxidase; coenzymes or prosthetic groups such as FAD, FMN, ATP, biotin, and heme; fluorescent labels such as fluorescein derivatives (fluorescein isothiocyanate (FITC), fluorescein thiofulbamil, and the like), rhodamine derivatives (tetramethylrhodamine, trimethylrhodamine (RITC), Texas Red, Rhodamine 110, and the like), Cy dyes (Cy3, Cy5, Cy5.5, and Cy7), Cy-chromium, spectrum green, spectrum orange, propidium iodide, allophycocyanin (APC), and R-phycoerythrin (R-PE); bioluminescent labels such as luciferase; chemiluminescent labels including luminol derivatives such as luminol, isoluminol, N-(4-aminobutyl)-N-ethylisoluminose ester, acridinium derivatives such as N-methylacridinium ester and N-methylacridinium acylsulfonamide ester, lucigenin, adamantyl dioxetane, indoxyl derivatives, and ruthenium complexes; and metals such as colloidal gold, which are detectable labels.

As described above, the composition of the present invention can be used for detecting (diagnosing) cancer and can also be used as it is for treatment. In other words, the present invention includes a method for cancer detection/diagnosis and treatment, which comprises administering the composition of the present invention to a subject, detecting/diagnosing a location and size of cancer in the subject using fluorescence emitted by IR700 in the administered composition, and killing the detected/diagnosed cancer by irradiating it with near-infrared light. The present invention may also be a composition used for both detecting and treating cancer (a detection and treatment composition), which contains the complex as an active ingredient.

In addition to the above, the present invention relates to the cancer detection composition, cancer treatment composition, or use of the complex of the present invention for producing the cancer detection and treatment composition. The present invention also relates to the complex of the present invention for cancer detection, cancer treatment, or cancer detection and treatment.

The composition of the present invention may be administered in any oral or parenteral formulation as long as it can reach the target cancer. In addition to intravenous administration, parenteral administration includes, for example, a method of spraying administration into the gastrointestinal mucosa through a forceps hole under endoscopic observation. The composition of the present invention is preferably for medical use, and therefore, it is preferably a formulation suitable for medical use. Examples of the composition for parenteral administration include injections, nasal drops, and suppositories. They are preferably injections (liquid or lyophilized preparations such as intravenous injections, subcutaneous injections, intradermal injections, intramuscular injections, intravitreal injections, and drip injections). Examples of aqueous liquids for injection include: buffers such as phosphates; and isotonic agents such as physiological saline, glucose, sucrose, mannitol, sodium chloride, and glycerin, to which additives, for example, solubilizing agents such as alcohols (e.g., ethanol), polyalcohols (e.g., propylene glycol, polyethylene glycol), nonionic surfactants (e.g., Polysorbate 80, Polysorbate 20, HCO-50 (polyoxyethylene (50 mol) adduct of hydrogenated castor oil)), and ethylenediamine, stabilizers such as sulfite, preservatives such as phenol, and pain relief agents such as lidocaine, can be added. For example, sesame oil, soybean oil, and the like can be used as oily liquids, to which benzyl benzoate, benzyl alcohol, and the like can be added as a solubilizing agent (see, if necessary: "Japanese Pharmaceutical Excipients Directory (JPED)" from Yakuji Nippo, Limited and "Handbook of Pharmaceutical Excipients Fifth Edition" from APhA Publications). Injections can be prepared according to known methods, for example, by dissolving, suspending, or emulsifying the complex in a sterile aqueous or oily liquid commonly used for injections. In the case of a lyophilized preparation, it can also be dissolved in water for injection, physiological saline, or the like so as to prepare an injection solution before use. Further, when the composition of the present invention is used as an injection, examples of storage containers can include ampoules, vials, prefilled syringes, cartridges for pen-type syringes, and bags for intravenous drips. Oral administration of proteins is generally considered difficult because the digestive system degrades them; however, oral administration may be possible, such as when targeting cancer of the digestive system. Examples of preparations for oral administration include capsules, tablets, syrups, and granules.

The composition of the present invention is suitably formulated in a dosage unit form that corresponds to the dose of the active ingredient. Examples of the dosage unit form include injections (ampoules, vials, and prefilled syringes). Each dosage unit form may typically contain from 1 to 1000 mg, from 5 to 250 mg, or from 10 to 100 mg of the complex of the present invention.

The composition of the present invention may be locally or systemically administered. There are no particular restrictions on the method of administration, and as mentioned above, it is administered parenterally or orally. Examples of parenteral administration routes include: intraocular, subcutaneous, and intraperitoneal injections or infusions; and injections or infusions into the blood (intravenous or intraarterial) or spinal fluid. Administration into the blood is preferable. The composition of the present invention may be administered only once or a plurality of times and may be temporary, continuous, or intermittent. Preferably, administration is carried out one to several times over a short period (within a few hours, within a few days, within 1 to 2 weeks, or the like). For example, in a case in which cancer cannot be treated entirely with one PIT treatment, PIT treatment for cancer can be performed a plurality of times at different intervals. In this case, the composition is administered one to several times before near-infrared irradiation for each treatment. Moreover, the complex of the present invention may be administered singly or in combination with other drugs.

The dose of the composition of the present invention is not particularly limited as long as it provides the desired treatment effect or detection/diagnosis effect and can be appropriately determined depending on symptoms, gender, age, and the like. The dose of the composition of the present invention can be determined using, for example, the treatment effect on cancer as an index. For example, when used for cancer treatment, a single dose of the active ingredient of the composition of the present invention may be from 0.01 to 20 mg/kg body weight, from 0.1 to 10 mg/kg body weight, or from 0.1 to 5 mg/kg body weight by intravenous injection once to several times. Similar amounts can be administered in other parenteral and oral administration cases. In the cases of particularly severe symptoms, the dose or frequency of administration (number of treatments) may be increased depending on the symptoms.

### Examples

Hereinafter, the present invention will be explained more specifically based on Examples. However, the present invention is not limited to these examples. All literature cited throughout the present application is incorporated herein by reference in their entirety.

### (Example 1) Preparation of IR700-Labeled Mouse Anti-c-KIT Antibody (whole body IgG antibody = hereinafter referred to as "whole IgG") and F(ab')₂ and Fab Fragments thereof

### (1) Preparation of Antigen

Human c-KIT (Accession No. NM-000222.3) was used as a target molecule for GIST. The antigen was prepared by incorporating a cDNA encoding a fusion protein (c-KIT-Fc) of the extracellular region (amino acids 1 to 504) of the c-KIT gene and the constant region (Fc portion) of mouse IgG2a into an expression vector (pcDNA3.1) according to an ordinary method (e.g., "Experimental Manual for Molecular Cell Biology," Nankodo Co., Ltd.) and further transfecting the recombinant vector into Chinese hamster ovary (CHO) cells. c-KIT-Fc-expressing CHO cells were cultured in a serum-free medium, namely TIL medium (Immuno-Biological Laboratories Co, Ltd.), and then purified from the culture supernatant using a Protein A column (GE Healthcare).

### (2) Preparation of Monoclonal Antibody

After BALB/c mice were immunized four times with the antigen prepared in (1), splenic mononuclear cells and fusion partner cells X63-Ag8-653 were fused with polyethylene glycol. Hybridomas were selected by the method described in J. Immunol. 146:3721-3728. Selection was performed by cloning cells producing antibodies, which react with immobilized c-KIT-Fc, and hybridoma 12A8 was isolated. Clone 12A8 produced IgG1 in the culture supernatant, and the antibody showed a strong reaction with the small cell lung cancer (SCLC) cell line SY and the GIST cell line GIST-T1, which express c-KIT.

### (3) Antigenic Determinant (Epitope) of Mouse Anti-c-KIT Antibody 12A8

The antigen-determining region recognized by the 12A8 antibody was determined by epitope mapping. A gene encoding a fusion protein of a different deletion mutant with a deletion from the extracellular region (amino acids 1 to 504) of human c-KIT and the C-terminus of the region and the constant region of mouse IgG2a was prepared and incorporated into an expression vector (pcDNA3.1). The prepared deletion mutants encode human c-KIT1-106 (c-KIT106-Fc), c-KIT1-205 (c-KIT205-Fc), c-KIT1-305 (c-KIT305-Fc), and c-KIT1-406 (c-KIT406-Fc) amino acids.

The expression vectors carrying these genes were each transfected into COS1 cells (ATCC, CRL-1650), and after culturing for two days in serum-free Dulbecco's MEM (Immuno-Biological Laboratories Co., Ltd.), the culture supernatant was collected. The collected culture supernatant was concentrated by ultrafiltration to a 10-fold concentration and dot-blotted onto a PVDF membrane (Millipore Co., Ltd., IPVH00010). The blotted PVDF membrane was subjected to blocking treatment, then reacted with a mouse anti-c-KIT (12A8) antibody, and further reacted with an anti-mouse Igx chain HRP-labeled antibody (Southern Biotech, 1050-05). Detection was performed by chemiluminescence detection using Amersham ECL (Cytiva, RPN2106).

Since all of the various dotted fusion proteins contained mouse IgG2aFc, they all showed positive for the anti-mouse IgG antibody (a-Mouse HRP) but no reactivity to the anti-mouse Igx chain HRP (a-mouse kappa HRP). The mouse anti-c-KIT (12A8) antibody (a-c-KIT 12A8) reacted only with c-KIT504-Fc without the C-terminal deletion used as the antigen but did not react with the other C-terminal deletion mutant fusion proteins. This clarified that an epitope exists in the region of amino acids 407 to 504, which is the extracellular region of c-KIT.

### (4) Production of Antibody

The 12A8 hybridoma was cultured in a high-density culture flask CELLINE CL-1000 (Argos BMA-90005) in serum-free TIL medium (Immuno-Biological Laboratories Co., Ltd.) in a CO₂ incubator at 37°C. The culture supernatant was purified using a Protein A column, thereby obtaining the 12A8 (IgG1) antibody.

### (5) Degradation of Antibody

The obtained 12A8 antibody was adjusted with 0.1 M/L sodium acetate buffer (pH 3.8) and digested with pepsin at room temperature for 2 hours and then purified by gel filtration chromatography (Ultrogel AcA44 column), thereby obtaining 12A8 F(ab')₂.

The 12A8 antibody was adjusted with 0.1 M/L sodium acetate buffer (Ph5.5), digested with papain for 3 hours at room temperature, then purified by gel filtration chromatography (Ultrogel AcA44 column), and further passed through a Protein A column to remove the Fc fragment, thereby obtaining Fab.

### (6) IR-700 Labeling

The antibody was labeled with IRDye700 using an IRDye700DX protein labeling kit (LI-COR Biosciences, Lincoln, NE) according to the manufacturer's instructions. The molecular weights of the whole IgG, F(ab')₂, and Fab of the 12A8 antibody were assumed to be 150,000, 110,000, and 50,000, respectively. The antibody and IRDye700 were mixed at a molar ratio of 1:5 and incubated at room temperature for 2 hours for conjugation therebetween. The amount of antibody was determined at an absorbance of 280 nm, and the amount of dye was determined at an absorbance of 689 nm. Then, F/P was calculated.

As a result, the F/P of each IR700-12A8 conjugate was 2.4 for whole IgG, 2.6 for F(ab')₂, 1.9 for Fab, and 3.8 for negative control IgG1 (not reactive to c-KIT).

Each conjugate was adjusted with a dilution buffer (PBS) such that the molar concentration at the time of use was the same (0.05 nM (Table 1) for the *in vitro* test, 0.5 nM (Table 2) for the *in vivo* test).

### (Example 2) In Vitro Binding of IR700-Labeled Mouse Anti-c-KIT Antibody and Fragment Thereof to GIST and Fluorescence Intensity Thereof

Live cell imaging of the GIST-T1 cell line was performed using the IR700-labeled mouse anti-c-KIT antibody (whole body IgG antibody = hereinafter referred to as "whole IgG") and F(ab')₂ and Fab fragments thereof prepared in Example 1 so as to confirm whether there was a difference in fluorescence concentration.

**[Table 1]**

| | IR700-labeled sample | F/P ratio | Concentration | Molecular weight |
|---|---|---|---|---|
| A | IR700-12A8 (whole IgG) | 2.4 | 0.80mg/ml | 150,000 |
| B | IR700-12A8 (Fab) | 1.9 | 1.45mg/ml | 50,000 |
| C | IR700-12A8 (F(ab')2) | 2.6 | 1.58mg/ml | 110,000 |
| D | IR700-IgG1 (negative control) | 3.8 | 0.30mg/ml | 150,000 |

Each of the obtained IR700-labeled binding antibodies was dispensed into small quantities and stored at -20°C until use.

RPMI RPMI 1640 medium (phenol red-free) (43 mL) to which 1.25 mL of 1M HEPES and 5 mL of FBS were added was used as the medium.

The GIST cultured cell line GIST-T1 (Dr. Tguchi T., Kouchi University, Kouchi, Japan) was cultured in a 10-cm dish (Greiner Bio-One International GmbH, CELLSTAR 664160-013) with the addition of the above-described RPMI1640 medium for proliferation.

GIST-T1 cells (8 × 10⁴ cells/dish) were seeded on five glass bottom dishes (Greiner Bio-One International GmbH, CELL view 627965) and cultured for 24 hours. After the supernatant was removed, the cells were incubated at room temperature for 15 minutes with the addition of 1 mL of blocking buffer (Dako, Protein Block Serum-Free, X0909). Each IR700-labeled sample listed in Table 1 was adjusted to a working concentration using an antibody dilution buffer (PBS containing 0.1% BSA). After 15 minutes, the blocking buffer was discarded, and 500 µL each of the 12A8 conjugate and a control conjugate (IR700-IgG1) were added. Among the IR700-12A8 labeled samples, whole IgG and F(ab')₂ were set at a final concentration of 0.05 nM; however, Fab with a monovalent antigen binding site was set at a two-fold final concentration of 0.1 nM so as to match the number of binding sites. Further, 5 µL of Hoechst 33342 solution (1 mg/mL, DOJINCO H342) was added to each of them and incubated on ice for 1 hour.

Thereafter, the antibody dilution buffer containing the IR700-labeled sample was removed, and after washing three times with the antibody dilution buffer, 1 mL of PBS was added to each of them. Fluorescence was observed using a confocal microscope (Keyence) at the following wavelengths:
IR700 λₑₓ: 620 nm, λₑₘ: 700 nm;
Hoechst 33342 λₑₓ: 360 nm, λₑₘ: 460 nm.

As shown in FIG. 1, each antibody of IR700-12A8 showed strong fluorescence intensity, but no reaction was observed with the negative target IR700-IgG1. A graph showing the fluorescence intensity measured in FIG. 1 is shown in FIG. 2. The 12A8 fragment F(ab')₂ showed similar fluorescence intensity to whole IgG in cell imaging and the 12A8 fragment Fab showed superior fluorescence intensity to whole IgG in cell imaging.

### (Example 3) Fluorescence Imaging of Mouse Xenograft Tumor Using 12A8 Fragment (in vivo test)

An immunosuppressant, namely cyclophosphamide (0.2 mg/g body weight), was administered to 10 athymic BALB/c nude mice (CLEA Japan Inc., Tokyo, Japan). GIST-T1 cells were adjusted to 1 × 10⁷ cells/50 µL in PBS and suspended in thawed Matrigel (Corning Incorporated, USA) at a ratio of 1:1 on ice. Two days after administration of the immunosuppressant, 100 µL of suspended GIST-T1 cells were subcutaneously transplanted into the right thigh of each nude mouse treated with the immunosuppressant. When the tumor grew to 8 mm, each antibody was administered and observed.

On Day 28 of transplantation, before antibody administration, autofluorescence was observed using IVIS Spectrum (Perkin Elmer Inc., Waltham MA) with excitation at 675 nm and emission at 720 nm.

Next, three types of labeled antibodies or labeled antibody fragments, IR700-12A8 (whole IgG), IR700-12A8 (Fab), and IR700-12A8 (F(ab')₂), were administered through the tail vein at 60 µg, 40 µg, and 44 µg, respectively. After 1 hour, 2 hours, 3 hours, 6 hours, 9 hours, 12 hours, 24 hours, and 48 hours, fluorescence observation was performed using IVIS with excitation at 675 nm and emission at 720 nm.

The results are shown in FIGS. 3 and 4. The 12A8 fragment (Fab, F(ab')₂) antibody showed earlier tumor accumulation and metabolism at 1, 2, and 3 hours after administration compared to whole IgG in the *in vivo* model. The peak value of fluorescence intensity in the *in vivo* model tended to be higher than that of whole IgG.

The signal-to-noise ratio (S/N ratio) in the *in vivo* model is shown in FIG. 5. The S/N ratio of the 12A8 fragment (Fab, F(ab')₂) was comparable to that of whole IgG. These results showed that by using a fragment of a GIST-specific antibody (12A8) bound to a fluorescent dye, it is possible to diagnose GIST within a short period (within 3 hours) after administering the antibody to a living body.

**[Table 2]**

| | IR700-labeled sample | F/P ratio | Concentration | Molecular weight |
|---|---|---|---|---|
| A | IR700-12A8 (whole IgG) | 2.3 | 0.69mg/ml | 150,000 |
| B | IR700-12A8 (Fab) | 1.9 | 1.45mg/ml | 50,000 |
| C | IR700-12A8 (F(ab')2) | 2.6 | 1.58mg/ml | 110,000 |

### (Example 4) In Vivo Photoimmunotherapy (PIT) Treatment Test

Twenty-eight days after GIST-T1 was transplanted into nude mice treated with the immunosuppressant, four mice having similar tumor sizes were selected. The mice were sedated with isoflavone, and IR700-12A8Fab and IR700-12A8F(ab')₂ were administered at 79 µg and 66 µg, respectively, through the tail vein, and about 2 hours later, the tumor was irradiated with a laser for 1 hour (735 J). A schematic diagram of the experimental scheme is shown in FIG. 6. The tumor size was observed for four weeks. On Day 55 after transplantation, a mouse anti-c-KIT antibody labeled with Alexa Fruor 647 (BioLegend Inc., San Diego, USA) was administered through the tail vein, and 48 hours later, the tumor was observed by IVIS.

As a result, as shown in FIG. 7, the tumor size in both the negative controls, Fab (vehicle) and F(ab')₂ (vehicle) mice, was the size of an index finger head. In mice treated with 735J laser irradiation, both IR700-12A8 Fab and F(ab')₂ tumors disappeared.

As a result of observation using IVIS, as shown in FIGS. 8 and 9, the control mice emitted intense fluorescence, but no fluorescence was observed in the mice subjected to PIT. It was found that by combining the fragment (Fab, F(ab')₂) of the GIST-specific antibody 12A8 with the dye IR700, the intractable disease, GIST, can be diagnosed at an early stage, and treatment can be started early after antibody administration, allowing tumors to be eliminated in a short period (FIGS. 7 to 9).

### (Example 5) Preparation of IR700-Humanized Anti-c-KIT (12A8) Antibody (whole IgG and Fab)

To safely use the mouse-derived anti-c-KIT monoclonal antibody 12A8 clone as a diagnostic composition and/or treatment composition for human gastrointestinal stromal tumor (GIST), the gene sequence of the mouse-derived anti-c-KIT monoclonal antibody 12A8 clone was replaced with a humanized antibody gene. The specific method is as follows.

### (1) Synthesis of Humanized Gene

A homology search for the variable region of the mouse anti-c-KIT (12A8) antibody gene sequence was performed on the human antibody gene database (NBCT). As a result, the heavy chain (HC) had high homology with human IGHV1-3, and the light chain (LC) had high homology with the human sequence IGKV2-29. Accordingly, while retaining the complementarity determining region (CDR) of the mouse anti-c-KIT (12A8) antibody gene sequence, the other frameworks were replaced with highly homologous human sequences, thereby constructing the variable region heavy chain (VH) and light chain (VL) sequences of the humanized anti-c-KIT (12A8) antibody. Artificial gene synthesis was performed from this gene sequence information, thereby creating VH and VL genes.

### (2) Construction of Expression Vector

Using the variable region genes (VH and VL) of the synthesized humanized anti-c-KIT (12A8) antibody and the natural human IgG1 gene, the heavy chain (HC) (FIG. 10, SEQ ID NO: 18) and light chain (LC) (FIG. 11, SEQ ID NO: 20) of the humanized antibody gene were created by overlap polymerase chain reaction (PCR).

HC fused the VH of the humanized anti-c-KIT (12A8) antibody with the human IgG constant region (Fc), and LC fused the VL of the humanized anti-c-KIT (12A8) antibody with the human Kappa chain constant region (KC).

For the use in expression of the Fab fragment, a gene with a stop codon inserted immediately before the hinge region (Fab HC) (FIG. 12, SEQ ID NO: 22) was amplified by PCR using the HC gene of the humanized anti-c-KIT (12A8) antibody as a template.

The HC gene, LC gene, and Fab HC gene of the humanized anti-c-KIT (12A8) antibody were each inserted into a mammalian cell expression vector (pCXN2-MCS, Immuno-Biological Laboratories Co, Ltd.) (FIG. 13), thereby preparing three types of antibody gene expression vectors: (i) humanized c-KIT (12A8) IgG1 (humanized c-KIT (12A8) IgG1) vector; (ii) humanized c-KIT (12A8) Igκ (humanized c-KIT (12A8)) Igκ) vector; and (iii) humanized c-KIT (12A8) Fab H (humanized c-KIT (12A8) Fab H) vector.

### (3) Expression by CHO-K1 Cells

To have a complete IgG1 (whole IgG) antibody of the humanized anti-c-KIT (12A8) antibody expressed, the following vectors were mixed and transfected into CHO-K1 cells using Lipofectamine 2000 (Thermo Fisher Scientific) according to the manual: (i) humanized c-KIT (12A8) IgG1 vector; and (ii) humanized c-KIT (12A8) Igκ vector. In addition, to have the Fab fragment of the humanized anti-c-KIT (12A8) antibody expressed, the following vectors were mixed and transfected into CHO-K1 cells using Lipofectamine 2000: (ii) humanized c-KIT (12A8) Igκ vector; and (iii) humanized c-KIT (12A8) Fab H vector.

A medium supplemented with antibiotic G-418 (Merck) was used for culture. The obtained culture supernatant was subjected to enzyme immunoassay (EIA) and SDS polyacrylamide gel electrophoresis SDS-PAGE), and positive cells were selected. Then, positive colonies were cultured by a limiting dilution method, and positive clones were selected. The results are shown in FIGS. 14 and 15.

To have a complete IgG1 (whole IgG) antibody expressed, the culture supernatant of CHO-K1 cells obtained by mixing and transfecting the following vectors was confirmed using the horseradish peroxidase (HRP)-labeled anti-human IgG (Fc) antibody (Immuno-Biological Laboratories Co, Ltd. (IBL)) and the HRP-labeled anti-human Kappa antibody (SouthernBiotech): (i) humanized c-KIT (12A8) IgG1 vector; and (ii) humanized c-KIT (12A8) Igκ vector.

As a result, as shown in FIG. 14, the same band that reacted with both labeled antibodies in a non-reduced state was confirmed. This band is substantially the same size as the weight average molecular weight (Mw) of the whole IgG and corresponds to two heavy chain (H) molecules and two light chain (L) molecules. In the case of reduction treatment, the band reacting with the HRP-labeled anti-IgG (Fc) antibody corresponded to the Mw of the heavy chain (HC) of humanized c-KIT (12A8) IgG1, and it corresponded to the Mw of the light chain (LC) of humanized c-KIT (12A8) Igκ for the HRP-labeled anti-Kappa antibody.

In addition, to have the Fab fragment expressed, the culture supernatant of CHO-K1 cells obtained by mixing and transfecting the following vectors was confirmed using the HRP-labeled anti-human Fd antibody (SouthernBiotech) and the HRP-labeled anti-human Kappa antibody: (ii) humanized c-KIT (12A8) Igκ vector and (iii) humanized c-KIT (12A8) Fab H vector.

As a result, as shown in FIG. 15, a band corresponding to the Mw of the Fab fragment was confirmed in a non-reduced state for both the anti-Fd antibody, which recognizes the H chain, and the anti-Kappa antibody, which recognizes the L chain. Naturally, a high molecular weight band corresponding to whole IgG was not observed in either HRP-labeled antibody. In the case of reduction treatment, a band corresponding to the Mw of humanized c-KIT (12A8) Fab H could be confirmed with the HRP-labeled anti-Fd antibody, and a band corresponding to the Mw of the light chain (LC) of humanized c-KIT (12A8) Igκ could be confirmed for the HRP-labeled anti-Kappa antibody (FIG. 15).

Lane 3 in FIG. 15 was a whole IgG antibody prepared using the following vectors: (i) humanized c-KIT (12A8) IgG1 vector; and (ii) humanized c-KIT (12A8) Igκ vector. No molecules corresponding to the Mw of Fab could be confirmed in either the reduced or non-reduced state for the HRP-labeled anti-Fd antibody and HRP-labeled anti-Kappa antibody.

From the above, it was confirmed that the humanized anti-c-KIT (12A8) IgG1 antibody and the Fab fragment of the humanized anti-c-KIT (12A8) antibody (hereinafter also referred to as "humanized anti-c-KIT (12A8) Fab antibody") were prepared.

### (4) Confirmation by EIA

Since the antigen recognition site of the anti-c-KIT (12A8) antibody is the extracellular region of the human c-KIT protein, a fusion protein (c-KIT-Fc) of the c-KIT extracellular region and the Fc region of the mouse antibody was used as the antigen for EIA. A fusion product of the extracellular region of the human FLT4 protein and mouse antibody Fc (FLT4-Fc) was used as a negative control.

Each above was solid-phased in a 96-well plate, blocked with a BSA solution, washed, sufficiently dried, and then stored in a refrigerator until use. The humanized anti-c-KIT (12A8) IgG1 antibody-producing clone culture supernatant was diluted 2-fold to 64-fold and added to an antigen solid-phase plate and a negative control plate so as to perform a primary reaction. The same was performed for the humanized anti-c-KIT (12A8) Fab antibody. The primary reaction was carried out for one hour at room temperature, and then each well was washed with phosphate-buffered saline (PBS). The HRP-labeled anti-human Kappa chain antibody was added as a secondary antibody and reacted for 30 minutes at room temperature. Thereafter, each well was washed with PBS, and the substrate OPD (Sigma-Aldrich) was added to develop a color. 1N sulfuric acid was added to stop the reaction, and the absorbance at 490 nm was measured.

As a result, as shown in FIG. 16, both the humanized anti-c-KIT (12A8) IgG1 antibody and the humanized anti-c-KIT (12A8) Fab antibody reacted with the antigen c-KIT-FC, but not with the negative control FLT4-Fc. Further, no reaction was observed in the negative control using MOCK (empty vector) expressed in CHO-K1.

The above confirmed that the humanized anti-c-KIT (12A8) IgG1 antibody and the humanized anti-c-KIT (12A8) Fab antibody have antigen specificity. To use each culture supernatant for *in vitro* and *in vivo* tests, the humanized anti-c-KIT (12A8) IgG1 antibody was purified using a protein A column, and the humanized anti-c-KIT (12A8) Fab antibody was purified using Kan Cap G (KANEKA CORPORATION).

### (Example 6) Examination of Reactivity of IR700-Humanized Anti-c-KIT (12A8) Antibody (whole IgG and Fab) by Flow Cytometry (in vitro)

Flow cytometry was performed using the IR700-labeled humanized anti-c-KIT (12A8) antibody (whole IgG and Fab) and the GIST-T1 cell line, thereby confirming the reactivity of the humanized antibody.

### (1) Preparation of GIST Cells

GIST-T1 cells were collected with trypsin (on ice), and 1 × 10⁶ cells were dispensed into 1.5 mL tubes. The cells were washed three times with 1 mL of PBS-BSA-Azide. To each tube, 1 mL of 4% paraformaldehyde cooled to 4°C was added and mixed by gentle pipetting on ice. The tubes were returned to room temperature and left to stand for 15 minutes (the subsequent process was carried out at room temperature). The cells were washed three times with 1 mL of PBS-BSA-Azide.

### (2) Reaction of Antibody

Various antibodies were labeled with IR700, and the following antibodies obtained were used. In FIG. 18, the IR700-labeled mouse anti-c-KIT (12A8) antibody is also referred to as 12A8 (Whole IgG), the IR700-labeled humanized anti-c-KIT (12A8) IgG1 antibody is also referred to as Humanized Whole IgG, and the IR700-labeled humanized anti-c-KIT (12A8) Fab antibody is also referred to as Humanized Fab.

**[Table 3]**

| IR700-labeled antibody | F/P ratio | Concentration | Molecular weight |
|---|---|---|---|
| IR700-labeled mouse anti-c-KIT (12A8) antibody | 4.4 | 0.30mg/ml | 150,000 |
| IR700-labeled humanized anti-c-KIT (12A8) IgG1 antibody | 2.0 | 0.826 mg/ml | 150,000 |
| IR700-labeled humanized anti-c-KIT (12A8) Fab antibody | 2.2 | 0.536 mg/ml | 50,000 |

To GIST-T1 cell pellets, 100 µL of each antibody solution prepared with PBS-BSA-Azide was added and mixed. The mixtures were prepared to contain 10 µg of the IR700-labeled mouse anti-c-KIT (12A8) antibody, 10µg of the IR700-labeled humanized anti-c-KIT (12A8) IgG1 antibody, and 10µg of the IR700-labeled humanized anti-c-KIT (12A8) Fab antibody, respectively. Each mixture was covered with aluminum foil in the dark and allowed to react at room temperature (from 20°C to 25°C) for 60 minutes (the subsequent process was carried out in the dark as much as possible). Washing was performed three times by adding 1 mL of PBS-BSA-Azide. After adding 500 µL of PBS-BSA-Azide and mixing, each mixture was passed through a filter (42-µm nylon mesh, AS ONE Corporation). Measurement was performed using FACS (CytoFLEX (Beckman Coulter)) with excitation at 638 nm and 712/25BP. The results are shown in FIG. 17. Here, 10 µg of the IR700-labeled humanized anti-c-KIT (12A8) Fab antibody was added; however, to match the amount of IR700 added with other antibodies, it was necessary to add 6.67 µg of the antibody. When the amount added is 6.67 µg, the amount of IR700 added becomes about 2/3 times; thus, it is thought that the MFI also becomes about 2/3 times.

The MFI of the IR700-labeled humanized anti-c-KIT (12A8) IgG1 antibody was similar to that when using the IR700-labeled mouse anti-c-KIT (12A8) antibody. Given the fact that the IR700-labeled mouse anti-c-KIT (12A8) antibody has a high F/P ratio of 4.4, the IR700-labeled humanized anti-c-KIT (12A8) IgG1 antibody was shown to have favorable reactivity with c-KIT. Also, concerning the IR700-labeled humanized anti-c-KIT (12A8) Fab antibody, when the amount administered is the equivalent mole (6.67 µg), MFI is considered to be the same as that of the IR700-labeled humanized anti-c-KIT (12A8) IgG1 antibody, suggesting excellent reactivity. Based on the above results, an *in vivo* experiment using small animals was conducted.

### (Example 7) Fluorescence Imaging of Xenograft Tumors in Mice Using IR700-Labeled Humanized Anti-c-KIT (12A8) Antibody (whole IgG and Fab)

### (1) Material

The IR700-labeled humanized anti-c-KIT (12A8) antibodies (whole IgG and Fab) used were the same as those used in Example 6.

### (2) Method

Immunosuppression treatment was performed by administering cyclophosphamide (0.2 mg/g body weight) to nine nude mice (CLEA Japan, Inc., BALB/cAJc1-nu/nu, 5 weeks old, female). Two days later, GIST-T1 prepared in a volume of 100 µL (cells: Matrigel = 50:50) and containing 1 × 10⁷ cells was transplanted into each of 9 mice. Specifically, Matrigel was thawed on ice, the cells were peeled off, the number of cells was counted, and the cells were suspended in PBS at 1 × 10⁷ cells/50 µL of GIST-T1 cells. On ice, cells were suspended in Matrigel at a cell solution: Matrigel ratio of 1: 1 and transplanted subcutaneously into the right thigh.

When the tumor size reached 8 mm, various antibodies were administered through the tail vein. Subsequently, immediately after administration, and 1 hour, 2 hours, 3 hours, 4 hours, 5 hours, 6 hours, 9 hours, 12 hours, and 24 hours after administration, fluorescence observation was performed using IVIS (λₑₓ: 675; λₑₘ: 720) (various antibodies: n = 3).

As a result, as shown in FIGS. 18 and 19, in fluorescence imaging after administering the IR700-labeled humanized anti-c-KIT (12A8) Fab antibody to mouse models *in vivo,* the fluorescence intensity in the tumor region (ROI) peaked after 3 to 4 hours and gradually decreased thereafter. On the other hand, the fluorescence intensity of the control ROI on the opposite side (left thigh) to which tumor cells were not administered peaked after 1 to 6 hours and gradually decreased thereafter. Further, in fluorescence imaging after administering the IR700-labeled humanized anti-c-KIT (12A8) IgG1 antibody, almost no fluorescence could be seen 1 to 4 hours after administration, and even though it could be seen, the fluorescence intensity was weak.

Furthermore, as shown in FIG. 20, the S/N ratio in the tumor region (ROI) after administering the IR700-labeled humanized anti-c-KIT (12A8) Fab antibody peaked 3 to 4 hours later and gradually decreased thereafter.

The above results showed that the IR700-labeled humanized anti-c-KIT (12A8) Fab antibody is allowed to accumulate in a tumor in a short period; thus, it is suitable for rapid diagnosis of GIST. Next, a treatment experiment was conducted using small animals.

### (Example 8) Photoimmunotherapy (in vivo) of Xenograft Tumors in Mice Using IR700-Labeled Humanized Anti-c-KIT (12A8) Fab Antibody

### (1) Material and Method

Among the mice that had undergone immunosuppressive treatment and GIST-T1 transplantation in the same manner as those used in Example 7, four mice with similar tumor sizes were selected and sedated with isoflurane. To the mice, 80 µg of the IR700-labeled humanized anti-c-KIT (12A8) Fab antibody was administered (n = 2), and about 3 hours later, the tumor in each mouse was irradiated with laser for 1 hour (735 J). Thereafter, the tumor size was observed for 3 weeks along with a control group (saline administration group) in which tumors were not treated. The treatment schedule is shown in FIG. 21. The Alexa Fluor 647-labeled anti-c-KIT antibody was administered through the tail vein and observed using IVIS 48 hours later (n = 2).

As a result, as shown in FIG. 22, the fluorescence intensity increased over time from 1 to 3 hours after administration of the IR700-labeled humanized anti-c-KIT (12A8) Fab antibody. As shown in FIG. 23, irradiation with a 685 nm laser 3 hours after administering the antibody showed that the tumor disappeared.

## Claims

1. A complex comprising: an antigen-binding fragment of the antibody having a complementarity determining region (CDR) in an antibody heavy chain consisting of an amino acid sequence set forth in SEQ ID NO: 2 or SEQ ID NO: 19; and a CDR in an antibody light chain consisting of an amino acid sequence set forth in SEQ ID NO: 4 or SEQ ID NO: 21; and IR700.

2. The complex according to claim 1, wherein the antigen-binding fragment of the antibody is Fab or F(ab')₂.

3. The complex according to claim 1 or 2, wherein the CDR in the antibody heavy chain having the amino acid sequence set forth in SEQ ID NO: 2 is the following (a) or (b):
(a) heavy chain CDR (CDRH) 1 consisting of an amino acid sequence set forth in SEQ ID NO: 7,
CDRH2 consisting of an amino acid sequence set forth in SEQ ID NO: 8, and
CDRH3 consisting of an amino acid sequence set forth in SEQ ID NO: 9; or
(b) CDRH1 consisting of an amino acid sequence set forth in SEQ ID NO: 13,
CDRH2 consisting of an amino acid sequence set forth in SEQ ID NO: 14, and
CDRH3 consisting of an amino acid sequence set forth in SEQ ID NO: 15.

4. The complex according to claim 3, wherein the CDR in the antibody light chain having the amino acid sequence set forth in SEQ ID NO: 4 is the following (a') or (b'):
(a') light chain CDR (CDRL) 1 consisting of an amino acid sequence set forth in SEQ ID NO: 10,
CDRL2 consisting of an amino acid sequence set forth in SEQ ID NO: 11, and
CDRL3 consisting of an amino acid sequence set forth in SEQ ID NO: 12; or
(b') CDRL1 consisting of an amino acid sequence set forth in SEQ ID NO: 16,
CDRL2 consisting of amino acid sequence GIS, and
CDRL3 consisting of an amino acid sequence set forth in SEQ ID NO: 17.

5. The complex according to any one of claims 1 to 4, wherein a number of IR700 molecules bound per molecule of the antigen-binding fragment of the antibody (F/P ratio) is from 1 to 10.

6. The complex according to any one of claims 1 to 5, wherein the antibody fragment is labeled by further being bound to another labeling substance.

7. A composition comprising a plurality of the complex according to any one of claims 1 to 6, wherein an average number of IR700 molecules bound per molecule of the antigen-binding fragment of the antibody of the complex in the composition (F/P ratio) is from 1 to 10.

8. A cancer detection composition containing the complex according to any one of claims 1 to 6 as an active ingredient.

9. The cancer detection composition according to claim 8, which is used for detecting cancer within 6 hours from administration of the composition.

10. A diagnostic composition containing the complex according to any one of claims 1 to 6 as an active ingredient.

11. The diagnostic composition according to claim 10, which is for cancer diagnosis.

12. A treatment composition containing the complex according to any one of claims 1 to 6 as an active ingredient.

13. The treatment composition according to claim 12, which is for cancer treatment.

14. The treatment composition according to claim 12 or 13, wherein after administering the composition, an area where the composition accumulates is irradiated with near-infrared light.

15. The treatment composition according to claim 14, wherein the composition is irradiated with near-infrared light within 1 to 12 hours after administration of the composition.

16. A composition used for both diagnosis and treatment containing the complex according to any one of claims 1 to 6 as an active ingredient.

17. The composition according to claim 16, which is for cancer diagnosis and treatment.
